# EUROPEAN PATENT SPECIFICATION

(11) **EP 2 968 755 B1**
(45) Date of publication and mention of the grant of the patent: **19.07.2017**
(21) Application number: 14716492.5
(22) Date of filing: 13.03.2014
(51) Int. Cl.: A61M 5/168

(54) **OPTICAL IMAGING SYSTEM WITH MULTIPLE IMAGING CHANNEL OPTICAL SENSING**
OPTISCHES BILDGEBUNGSSYSTEM MIT MEHRFACHBILDGEBUNGSKANALOPTIK
SYSTÈME D'IMAGERIE OPTIQUE PRÉSENTANT UNE OPTIQUE D'IMAGERIE À CANAUX MULTIPLE

(30) Priority: 14.03.2013 US 201313828744
(43) Date of publication of application: 20.01.2016
(60) Divisional application: 17175334.6
(73) Proprietor: Baxter International Inc, Deerfield, IL 60015 (US); Baxter Healthcare S.A., 8152 Glattpark (Opfikon) (CH)
(72) Inventor: MUNRO, James F., Ontario, New York 14519 (US)
(74) Representative: Potter Clarkson LLP
(86) International application number: PCT/US2014/026284
(87) International publication number: WO 2014/160307

(56) References cited:
- EP-A1- 0 569 728
- WO-A1-2006/077578
- US-A1- 2008 051 732
- US-A1- 2009 097 029
- US-A1- 2009 262 351
- US-A1- 2012 095 433

## Description

### TECHNICAL FIELD

The present disclosure relates generally to an infusion pump with chromatic multiplexing, in particular, the pump uses single or multiple light sources, a single lens, mirrors, and beam combiners to enable use of a single color image sensor to provide distinct images for multiple distinct portions of the pump.

### BACKGROUND

Monochrome image sensors are generally less costly than color image sensors. However, for simultaneously received multiple images, monochrome sensors cannot be used to separate the respective images, for example to generate, display, or operate upon the respective images, using conventional signal processing. For example, when a pixel in the monochrome sensor receives light, the sensor cannot determine which of the respective images the light pertains to.

US 2008/051732 describes drop sensing devices for monitoring intravenous fluid flow and more particularly to optical sensors for such drop sensing devices for reliably sensing drops passing through a drip chamber of an IV infusion set.

US 2012/095433 relates generally to a pump with optical imaging for calculating drop size and flow rate and for use in pump control and alarm operations.

US 2009/097029 relates to the use of an optical detection sensor that detects presence or absence of a product in a fluid delivery medium.

US 2009/262351 relates to an optical detection sensor that detects the presence or absence of a product in a fluid delivery medium. For example, in a fluid dispensing system in which one or more products are delivered, one or more such sensors could be utilized to detect presence or absence of product within the fluid delivery medium.

WO 2006/077578 describes an optical system for measuring low flow-rates of a fluid that passes a measuring point in the form of discrete drops.

EP 0 569 728 relates to methods of and/or apparatus for controlling the supply of a liquid and has been particularly devised for controlling the supply of water for use in humidifying apparatus.

### SUMMARY

According to aspects illustrated herein, there is provided an optical imaging system for use with an infusion tube having a drip chamber including a first portion with a drip tube, a second portion with an exit port, and a third portion located between the first and second portions, the optical imaging system including: at least one light source for emitting at least two of first, second, or third spectrums of light; an optics system including a single lens for receiving and transmitting at least two of the first spectrum of light transmitted through the first portion, the second spectrum of light transmitted through the second portion, or the third spectrum of light transmitted through the third portion. The optical system includes a single image sensor for receiving the at least two of the first, second, or third spectrums of light from the single lens and generating and transmitting data characterizing the at least two of the first, second, or third spectrums of light received from the single lens. The imaging system includes a memory element for storing computer executable instructions; and at least one processor configured to execute the computer executable instructions to generate, using the data, at least two of first, second, or third images of the first, second, or third portions, respectively.

According to aspects illustrated herein, there is provided an optical imaging system for use with an infusion tube having a drip chamber including a first portion with a drip tube, a second portion with an exit port, and a third portion located between the first and second portions, the optical imaging system including: a single light source for emitting at least two of first, second, or third spectrums of light; and an optics system including a single lens for receiving and transmitting at least two of: the first spectrum of light transmitted through the first portion; the second spectrum of light transmitted through the second portion; and the third spectrum of light transmitted through the third portion; and a single color image sensor for: receiving the at least two of the first, second, or third spectrums of light from the single lens; and generating and transmitting data characterizing the at least two of the first, second, or third spectrums of light received from the single lens. The imaging system includes a memory element for storing computer executable instructions, and at least one processor configured to execute the computer executable instructions to generate, using the data, at least two of first, second, or third images of the first, second, or third portions, respectively.

According to aspects illustrated herein, there is provided an optical imaging system for use with an infusion tube having a drip chamber including a first portion with a drip tube, a second portion with an exit port, and a third portion located between the first and second portions. The optical imaging system includes: at least one of a first light source for emitting a first spectrum of light only, a second light source for emitting a second spectrum of light only, or third source of light for emitting a third spectrum of light only; and an optics system including a single lens for receiving and transmitting at least one of: the first spectrum of light transmitted through the first portion; the second spectrum of light transmitted through the second portion; and the third spectrum of light transmitted through the third portion. The optical system includes a single color image sensor for receiving the at least one of the first, second, or third spectrums of light from the single lens and generating and transmitting data characterizing the at least one of the first, second, or third spectrums of light received from the single lens. The imaging system includes a memory element for storing computer executable instructions, and at least one processor configured to execute the computer executable instructions to generate, using the data, at least one of first, second, or third images of the first, second, or third portions, respectively. The first, second, and third spectrums of light are free of overlapping wavelengths amongst each other.

According to aspects illustrated herein, there is provided a method of imaging an infusion tube having a drip chamber including a first portion with a drip tube, a second portion with an exit port, and a third portion located between the first and second portions, including: storing, in a memory element, computer executable instructions; emitting at least two of first, second, or third spectrums of light from at least one light source; receiving and transmitting, using a single lens at least two of: the first spectrum of light transmitted through the first portion; the second spectrum of light transmitted through the second portion; or the third spectrum of light transmitted through the third portion; receiving, using a single image sensor, the at least two of the first, second, or third spectrums of light from the single lens; generating and transmitting, using the single image sensor data characterizing the at least two of the first, second, or third spectrums of light received from the single lens; and executing, using the at least one processor, the computer executable instructions to generate, using the data, at least two of first, second, or third images of the first, second, or third portions, respectively.

According to aspects illustrated herein, there is provided a method of imaging an infusion tube having a drip chamber including a first portion with a drip tube, a second portion with an exit port, and a third portion located between the first and second portions, including: storing computer executable instructions in a memory element; emitting, using a single light source, at least two of first, second, or third spectrums of light: receiving and transmitting, using a single lens at least two of: the first spectrum of light transmitted through the first portion; the second spectrum of light transmitted through the second portion; or the third spectrum of light transmitted through the third portion; receiving, using a single color image sensor, the at least two of the first, second, or third spectrums of light from the single lens; generating and transmitting, using a single color image sensor, data characterizing the at least two of the first, second, or third spectrums of light received from the single lens; and executing, using at least one processor, the computer executable instructions to generate, using the data, at least two of first, second, or third images of the first, second, or third portions, respectively.

According to aspects illustrated herein, there is provided a method of imaging an infusion tube having a drip chamber including a first portion with a drip tube, a second portion with an exit port, and a third portion located between the first and second portions, including: storing computer executable instructions in a memory element; and emitting at least one of a first spectrum of light only using a first light source, a second spectrum of light only using a second light source; or a third spectrum of light only using a third light source. The method includes: receiving and transmitting, using a single lens at least one of: the first spectrum of light transmitted through the first portion; the second spectrum of light transmitted through the second portion; or the third spectrum of light transmitted through the third portion; receiving, using a single color image sensor, the at least one of the first, second, or third spectrums of light from the single lens; generating and transmitting, using the single color image sensor, data characterizing the at least one of the first, second, or third spectrums of light received from the single lens; and executing, using at least one processor, the computer executable instructions to generate, using the data, at least one of first, second, or third images of the first, second, or third portions, respectively. The first, second, and third spectrums of light are free of overlapping wavelengths amongst each other.

### BRIEF DESCRIPTION OF THE DRAWINGS

The nature and mode of operation of the present invention will now be more fully described in the following detailed description of the invention taken with the accompanying drawing figures, in which:
Figure 1 is a schematic representation of definitions for an infusion pump;
Figure 2 is a schematic block representation of an infusion pump with an optical imaging system;
Figures 3A through 3F illustrate example embodiments of the illumination system shown in Figure 2;
Figures 4A through 4C are schematic representation of embodiments for an optical system;
Figures 5A through 5C illustrate imaging processing definitions;
Figure 6 illustrates an image of a drop including a circle at least partly included within an outer boundary of the drop
Figure 7 is a flow chart illustrating operation of a pump with an optical imaging system;
Figures 8A and 8B are schematic details for a pump implementing an operation for determining a gravity vector;
Figures 9A and 9B are schematic details of a pump using light injection;
Figures 10A and 10B are schematic details of a pump with a meniscus detection arrangement;
Figure 11 is a schematic block representation of two infusion pumps with respective optical imaging system in a primary and secondary configuration;
Figure 12 is a top-level block diagram illustrating operation of a pump with an optical imaging system;
Figure 13 is a block diagram illustrating example signal processing and feedback control for a pump with an optical imaging system;
Figure 14 is a block diagram illustrating example digital filtering in a pump with an optical imaging system;
Figure 15 is a schematic representation of example spatial filtering in a pump with an optical imaging system;
Figure 16 is a schematic representation of an optical imaging system with multiple imaging channel optical sensing and a single light source;
Figure 17 is a schematic representation of an optical imaging system with multiple imaging channel optical sensing and a single light source;
Figure 18 is a schematic representation of an optical imaging system with multiple imaging channel optical sensing and a single light source;
Figure 19 is a schematic representation of an optical imaging system with multiple imaging channel optical sensing and multiple light sources; and,
Figure 20 is a schematic representation of an optical imaging system with two-channel optical imaging and a single light source.

### DETAILED DESCRIPTION

At the outset, it should be appreciated that like drawing numbers on different drawing views identify identical, or functionally similar, structural elements of the invention. While the present invention is described with respect to what is presently considered to be the preferred aspects, it is to be understood that the invention as claimed is not limited to the disclosed aspects.

Furthermore, it is understood that this invention is not limited to the particular methodology, materials and modifications described and as such may, of course, vary. It is also understood that the terminology used herein is for the purpose of describing particular aspects only, and is not intended to limit the scope of the present invention, which is limited only by the appended claims.

Unless defined otherwise, all technical and scientific terms used herein have the same meaning as commonly understood to one of ordinary skill in the art to which this invention belongs. Although any methods, devices or materials similar or equivalent to those described herein can be used in the practice or testing of the invention, the preferred methods, devices, and materials are now described.

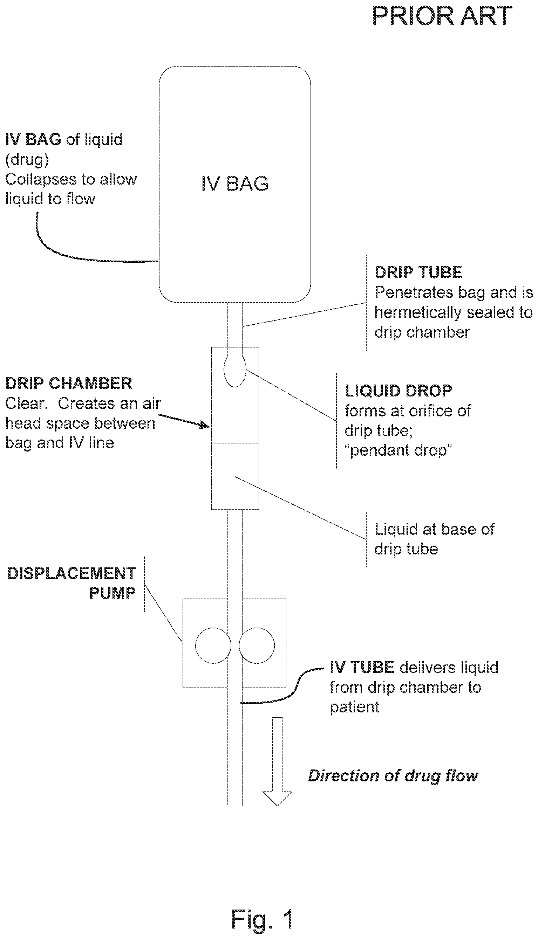

Figure 1 is a schematic representation of definitions for an infusion pump.

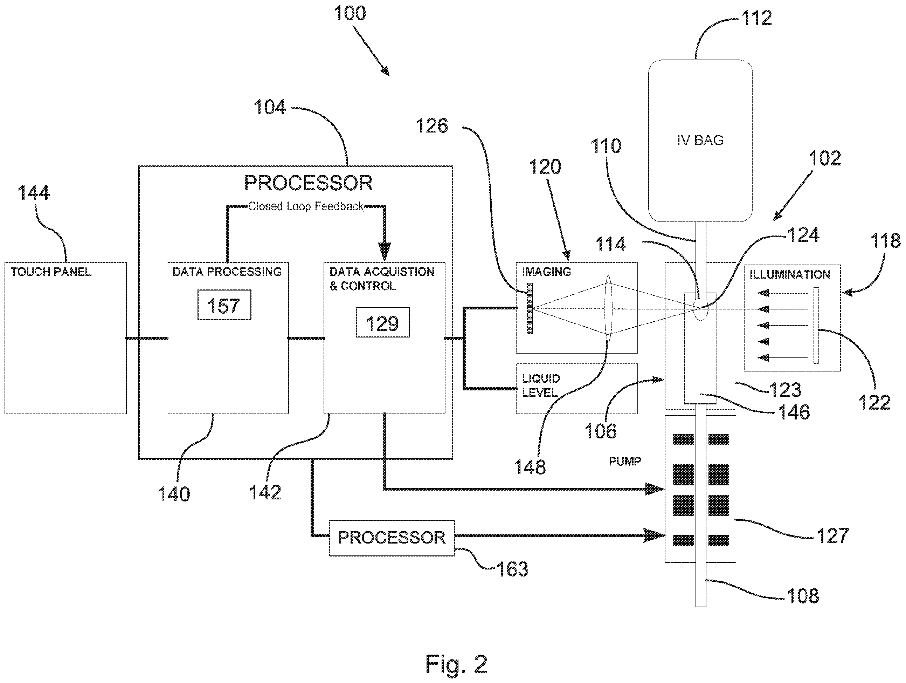

Figure 2 is a schematic block representation of infusion pump **100** with optical imaging system **102**. Pump **100** includes specially programmed microprocessor **104**, drip chamber **106** for connection to output tube **108**, and drip tube **110** for connecting the drip chamber to a source of fluid **112**, for example, an IV bag. The drip tube includes end **114** disposed within the drip chamber. The imaging system includes illumination system **118** and optical system **120**. System **118** includes lighting element **122** for transmitting light through wall **123** of the drip chamber to or around drop **124** of the fluid suspended from the end of the drip tube, for example, one or both of the drip and end **114** are illuminated. System **118** also controls illumination properties of the light transmitted to the drop. System **120** receives, for example using optical sensor **126**, light transmitted through the drop, or through or around end **114** and transmits, to the microprocessor, data **129** regarding the received light. Pump **100** also includes pumping mechanism **127.** In one embodiment, the mechanism includes top and bottom flow restrictors and uses peristaltic actuators, such as rollers, to displace fluid through tube **108**.

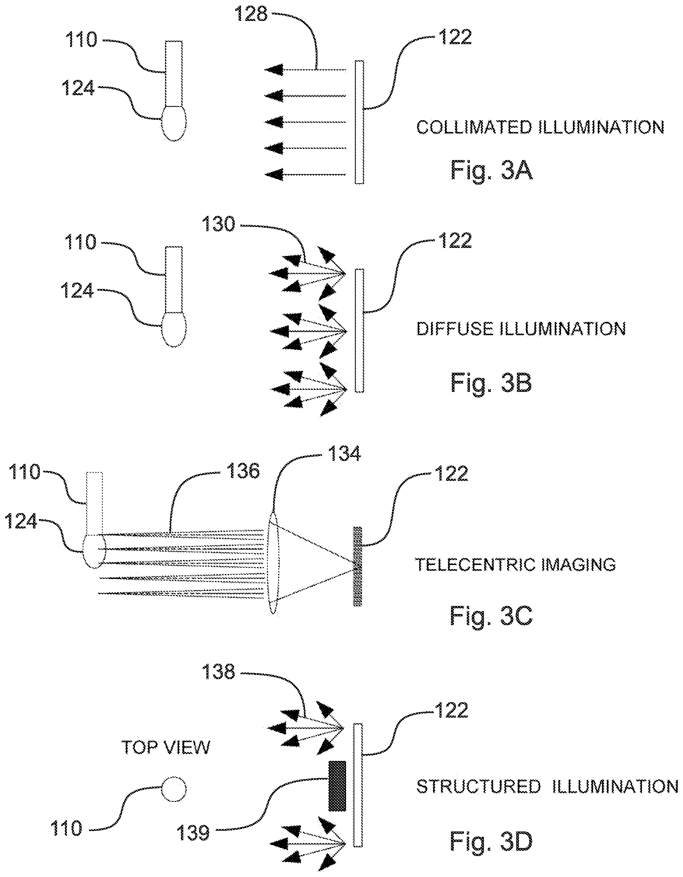

Figures 3A through 3F illustrate example embodiments of system **118** in Figure 2. As shown in Figure 3A, light rays **128** from a collimated illumination system are parallel. As shown in Figure 3B, light rays **130** from a diffuse illumination system are emitted in a cone-shaped pattern from each light emitting point on an illumination plane. As shown in Figure 3C, light rays **132** from illumination source **122** pass through telecentric lens **134** and are formed into ray bundles **136.** The rays in bundles **136** are very nearly parallel. The ray bundles provide sharp definition of image edges and minimize depth distortion As shown in Figure 3D, a structured lighting element shapes illumination, for example, rays **138**, so as to control unwanted or stray light and to accentuate edges of an objecting being illuminated. A structured lighting element can include barrier **139**, disposed between an illumination source and an object being illuminated, for example, drop **124**, to shape the illumination, for example, by blocking or altering light emanating from the source.

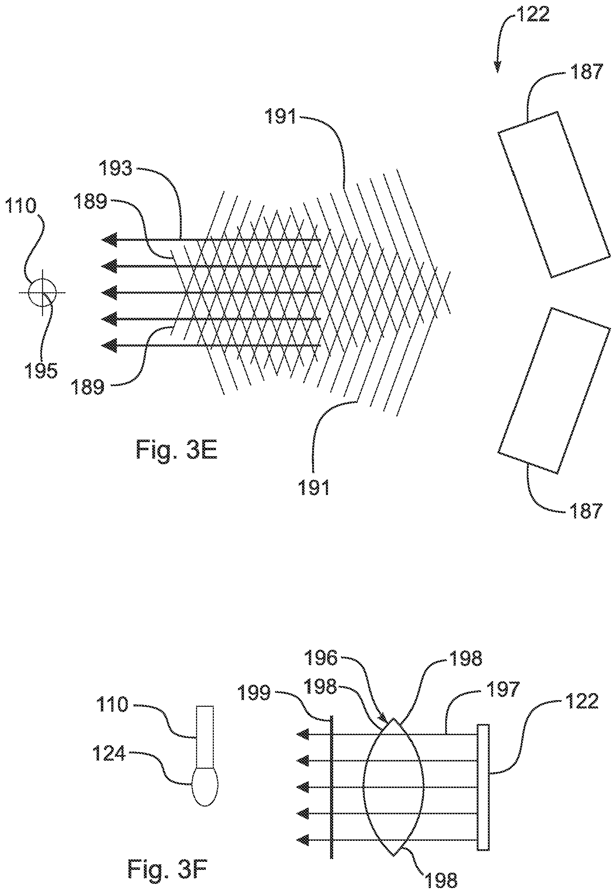

Figure 3E illustrates the use of laser interference to project stripe patterns measure drop **124**. Illumination source **122** includes laser light sources **187**. Sources **187** project light patterns consisting of many stripes at once, or of arbitrary fringes. This technique enables the acquisition of a multitude of samples regarding an image of drop **124**, simultaneously. As seen from different viewpoints, the projected pattern appears geometrically distorted due to the surface shape of the object. In one embodiment, patterns of parallel stripes are used; however, it should be understood that other patterns can be used. The displacement of the stripes allows for an exact retrieval of the three dimensional (3D) coordinates of details on an object's surface, for example, the surface of drop **124**. Laser interference works with two wide planar fronts **189** from laser beams **191**. The interference of the fronts results in regular, equidistant line, or interference, patterns **193**. Different pattern sizes can be obtained by changing the angle between the beams. The method allows for the exact and easy generation of very fine patterns with unlimited depth of field. Figure 3E is a top view of pump **100** and sources **187** are shown disposed radially about axis **195** for drop tube **110**. However, it should be understood that other configurations of sources **187** with respect to the pump are possible, for example, parallel to axis **195**.

Figure 3F illustrates the use of projection lens **196** in system **118**. In Figure 3F, system **118** illumination source transmits light **197** through lens **196**. Surface **198** of the lens is modified as known in the art, for example, etched or through deposition of chrome or other materials, to produce a pattern on the surface. Light **197** passing through the lens projects an image of the pattern on and about drop **124**. In one embodiment, projected pattern **199** is in the form of a constant-interval bar and space square wave, such as a Ronchi Ruling, or Ronchi grating.

The illumination source for a structured lighting element can be collimated, diffuse, or telecentric. Structured illumination can control unwanted or stray light and accentuate image edges. In one embodiment, the illumination system includes a telecentric lighting element. In one embodiment, the illumination system includes a structured lighting element.

Returning to Figure 2, microprocessor **104** includes data processing segment **140** and data acquisition and control segment **142**. The pump also includes control panel **144**, for example, any graphical user interface known in the art. Output from the optical system, for example, data **129** from sensor **126**, is inputted to segment **142**. Panel **144**, or other operator input, is used to input a desired flow rate through the drip chamber, as well as other necessary data such as drug type and treatment information. Microprocessor **104** can be any microprocessor known in the art.

Pump **100** uses optical sensing of pendant drops, that is drops hanging from or suspended from end **114**, to measure fluid flow through the drip chamber to the output tube and to provide input to a closed-loop pump control process controlled by the microprocessor. Fluid from source **112** flows through drip tube to end **114** of the drip tube. The fluid forms drop **124** at end **114** and when conditions in the drip tube, discussed infra, are suitable, the drop falls from end **114** into fluid **146** in the drip chamber. In general, a pendant drop increases in volume in proportion to the outflow of fluid **146** from the drip chamber through tube **108.** That is, an increase in the volume of the pendant drop during a time frame is equal to the volume of fluid passing from the drip chamber to tube **108** in the time period. The preceding relationship is based on the following assumptions: the fluid from the source is not compressible; source **112**, the drip tube, the drip chamber, tube **108**, and a patient to whom tube **108** is connected are closed to outside atmosphere. Each measurement of the drop volume is processed to provide a fluid volume (or mass) measurement. Successive measurements of drop volume over known intervals of time are used by the microprocessor to calculate the flow rate of fluid through the system.

Thus, in one embodiment, operation of pumping mechanism **127** is controlled by the microprocessor using the desired set point for flow through the drip chamber and data regarding a measured flow rate of fluid through the drip chamber. For example, the microprocessor executes a feedback loop which compares the desired flow rate with the measured flow rate, and adjusts the pumping mechanism to correct any deviations between desired and measured flow rates.

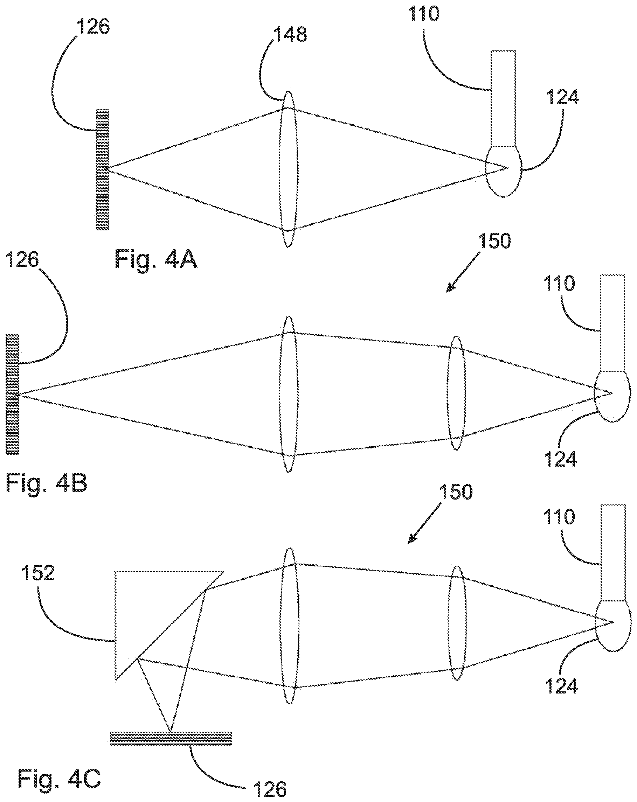

Figures 4A through 4C are schematic representation of embodiments for optical system **120**. The embodiments shown in Figures 4A through 4C form real, conjugate images, for example, of drop **124** on a focal plane array formed by sensor **126**. Figures 4A and 4B use refractive optics, such as single lens **148** or combinations **150** of lenses, respectively. Figure 4C shows refractive optics, such as combination **150** of lenses, and reflective optics, such as fold mirror **152**. Lens **148**, combination **150**, and mirror **152** can be any lens, combination of lenses, or mirror known in the art. Combination **150** may include different lenses in Figures 4B and 4C.

Returning to Figure 2, in one embodiment, optical sensor **126** is a focal plane array formed by any means known in the art, including, but not limited to a charge coupled device (CCD), a CMOS detector, or a hybrid imaging array such as InGaAs bonded to a CMOS readout integrated circuit. System **120** includes optics, such as lens **148**, focused on the location of drop **124**. It should be understood that other optics can be used in system **120**. In one embodiment, chamber **106** is substantially optically clear and system **118** directs light though the walls of the chamber to the optical system, for example, sensor **126**. The light can provide back or side illumination of the drop. In one embodiment, system **102** is configured such that drop **124** and the focal plane array are optical conjugates and the focal plane array records an actual image of the drop. The imaging system captures drop images at a rate sufficient to observe the growth and detachment of a single drop.

In one embodiment, pump **100** satisfies two key metrics with respect to imaging drop **124**. First, the frame rate (images per second) is sufficient to capture a sequence of images as the drop grows in size and detaches. Second, the exposure time (the amount of time the light is collected on the sensor for each specific image) is short enough to freeze the motion of the drop. Pump **100** generates images with clear edge definition, sufficient magnification (in terms of number of pixels across the drop), and a minimum number of artifacts such as glare.

In one embodiment, imaging system **102** and the microprocessor produce an accurate image of the drop that is then analyzed as described infra to determine the volume of the drop. Since the fluid drop has a uniform density, and any bubbles (occlusions) or entrainments are sufficiently small to be negligible, in one embodiment, only the outer surface of the drop is measured to calculate the volume of the drop. The preceding measurement is accomplished by imaging the drop with sufficient spatial resolution to accurately measure the boundary surface. A numeric integral over this boundary then provides the droplet volume.

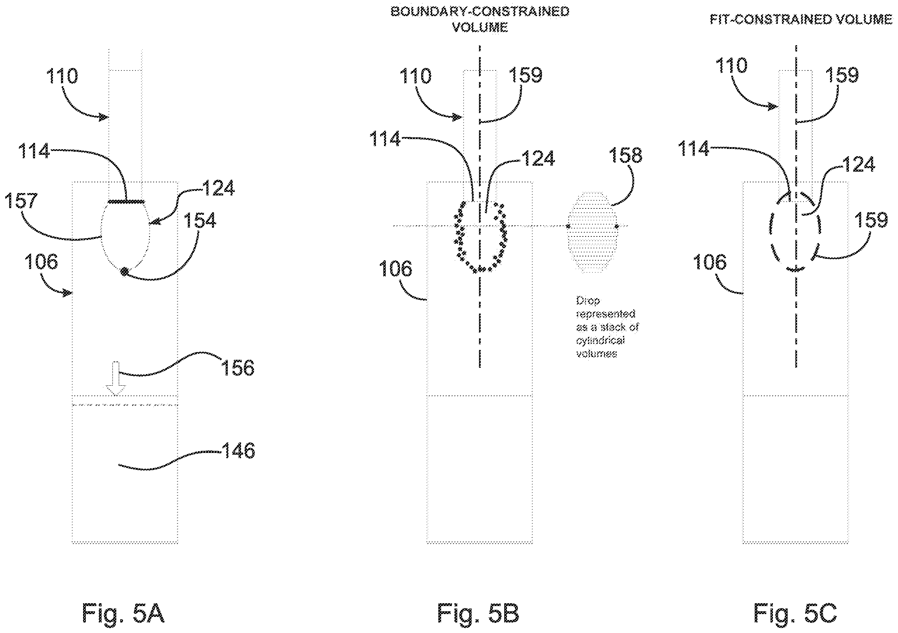

Figures 5A through 5C illustrate imaging processing definitions. In one embodiment, a reference/alignment frame and an image scale (pixels per mm) are established by locating end point **114** of the drip tube orifice, as shown in Figure 5A. The end point has a known size and hence provides scale calibration. The end point also represents the top boundary of the drop, which is used in volume calculations described infra. In one embodiment, apex **154** of the drop (a point furthest from the fixed/reference point) is identified and used in the determination of the volume of the drop. For example, the optical system, for example, sensor **126**, receives the light transmitted into or through the drip tube and transmitting, to the microprocessor, data regarding the received light. In one embodiment, the microprocessor is for determining, using the data, a boundary of end point **114** and using the boundary of end point **114** as a reference point for determining a volume, shape, or location of the drop, as further described infra.

In one embodiment, as further described infra, the direction of gravity (gravity vector **156**) with respect to drop **124** is determined. A reference point, for example, the boundary of end point **114**, and the gravity vector are used to establish a reference frame for the image processing.

In one embodiment, volume of drop **124** is calculated by using the microprocessor to receive data **129** and generate an image of the drop from the data. The microprocessor locates an outer edge of the drop in the image to define boundary **157** of the drop. The microprocessor integrates an area enclosed by the boundary and calculates a volume of revolution for the drop with respect to axis **159** for the drop that intersects the end of the drip tube, assuming symmetry of the drop with respect to the axis.

The above calculation of the volume of drip **124** can be calculated using at least two broad approaches. The first approach, termed Boundary Constrained Volume and shown in Figure 5B, uses the outer location of the drop image to calculate the total volume. Each horizontal row **158** of pixel data from the image has associated with it an outer left and right boundary. The area between these boundaries is treated as the two dimensional projection of a circular disk volume (the symmetric volume of rotation of the area). The drop image is integrated from end point **114** to the apex by summing the volume of each row. Boundary Constrained Volume obtains maximum resolution for each row of data.

The second approach is termed Fit Constrained Volume and is shown in Figure 5C. That is, the volume of drop **124** is determined by fitting a parametric function to the boundary image of the drop and integrating the parametric function, again, assuming rotational symmetry. There are a number of possible fitting algorithms, as discussed below, but the result of any fit is a set of parameters to the assumed function that represents entire boundary **157**. Fit Constrained Volume smoothes out row detail.

In one embodiment, the microprocessor creates a plurality of temporally successive images of the drop from data **129** and calculates a respective volume for the drop in each successive image or calculates respective time periods between detachment of successive drops from the end of the drip tube. By temporally successive images, we mean a series of images taken over a time period in chronological order. The microprocessor calculates a rate of increase for the volume of the drop using the respective volumes or the respective time periods. As noted above, flow out of the drip tube is substantially equal to the increase in the volume of the drop; therefore, the time periods between drops detaching from the end of the drip tube can be correlated to the volume increases of the successive drops. For example, in one embodiment, the microprocessor calculates a respective volume for the drop in each successive image, for example, using operations described infra and supra; calculates changes in the respective volumes; and calculates a flow rate of fluid to the output tube based on the changes in the respective volumes. In one embodiment, the microprocessor controls mechanism **127** to match the calculated flow rate with a desired flow rate, for example, stored in the microprocessor.

In one embodiment, the microprocessor is for generating a free flow alarm or an out of bound condition alarm when the rate of increase for the volume of the drops exceeds a predetermined value, for example, stored in the microprocessor. In one embodiment, the microprocessor is for operating mechanism **127** to shut off flow to the output tube when the free flow alarm or the out of bound condition alarm is generated. In one embodiment the microprocessor generates a downstream occlusion alarm when the rate of increase of the volume of the drop is less than a predetermined value. In one embodiment, the microprocessor determines that a drop is absent from the end of the drip tube for a specified period of time and generates an empty bag alarm or an air-in-line alarm.

In one embodiment, the pump includes processor **163** used to operate mechanism **127** to shut off flow to the output tube when the free flow alarm or the out of bound condition alarm is generated. That is, as a safety and redundancy factor, a second microprocessor is used in the pump.

The drop is initially hanging from a fixed point in the drip chamber, for example, end **114**. In one embodiment, the microprocessor is for identifying when the drop detaches from the fixed point in the drip chamber as a means of determining when the drop has reached maximum volume. The microprocessor makes the preceding identification by creating a plurality of temporally successive images of the drop and analyzing these images. By temporally successive images, we mean a series of images taken over a time period in chronological order.

In one embodiment, the microprocessor identifies, in each successive image, a respective point in the boundary, for example, apex **154**, and determines a distance of each respective point from end **114**. The microprocessor then identifies two successive images of the drop in which the distance, noted above, in the second image in the succession is less than the distance in the first image in the succession. This decrease of the distance indicates that the drop detached from the fixed point in the interval between the first and second images, which further indicates that the drop reached a maximum size in the first image. The microprocessor calculates the volume of the drop using the first image.

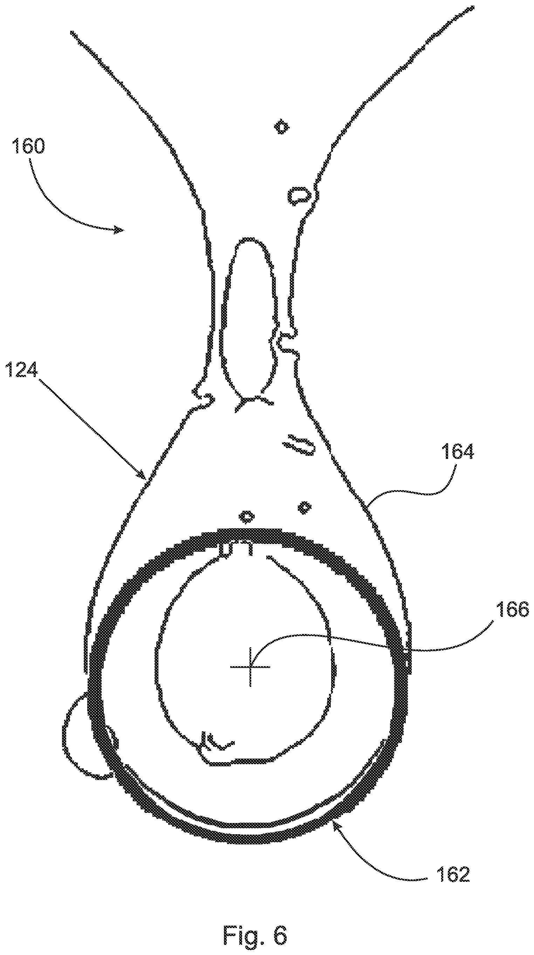

Figure 6 illustrates image **160** of drop **124** including circle **162** at least partly included within outer boundary **164** of the drop. Figure 6 illustrates a specific example of the Fit Constrained Volume approach. In one embodiment, the microprocessor identifies respective circles **162** within each temporally successive image. The circles are partially defined by a respective outer boundaries **164** of the temporally successive images. The microprocessor identifies a respective location, with respect to the fixed point in the drip chamber, for each respective circle and calculates a volume of the drop from the data and using the respective circles.

In one embodiment, identifying the respective location for said each respective circle includes identifying the image corresponding to the largest size of the drop, for example, the last image before the drop detaches from the end point of the drip tube. For example, the microprocessor identifies a respective point on each respective circle at a furthest distance from the fixed point in the drip chamber, for example, end point **114**. The microprocessor then determines which of the respective points is furthest from the fixed point and identifies an image including the respective point furthest from the fixed point. That is, the microprocessor identifies the largest drop by identifying the drop having the largest circle. In one embodiment, the largest drop is identified by determining a first image in which the distance of the apex from the fixed point decreases with respect to the distance of the apex from the fixed point for a second image immediately preceding the first image. This decrease indicates that the drop detached from the fixed point in the interval between the first and second images, which further indicates that the drop reached a maximum size in the first image. The microprocessor calculates the volume of the drop using the image including the respective point furthest from the fixed point.

In one embodiment, the microprocessor identifies the respective outer boundaries for each of the temporal images such that each outer boundary includes a respective edge of the drop furthest from the fixed point in the drip chamber and the respective circle includes the respective edge. That is, the microprocessor aligns the circles described supra with the actual edges of the drops such that the points of the circles furthest from the fixed point, for example, end **114**, are part of the edge of the drop. In one embodiment, the microprocessor identifies respective circular arcs corresponding to the respective edges and including the respective circular arcs in the respective circles.

In one embodiment, identifying the image corresponding to the largest size of the drop, for example, the last image before the drop detaches from the end point of the drip tube, includes using the center points of the circles. For example, the microprocessor calculates respective center points **166** for the circles and calculates the positions of the center points with respect to the fixed point, for example, end point **114**. The microprocessor then determines which of the center points is furthest from the fixed point and identifies an image including the center point furthest from the fixed point. That is, the microprocessor identifies the largest drop by identifying the drop having the largest circle. The microprocessor calculates the volume of the drop using the image including the center point furthest from the fixed point.

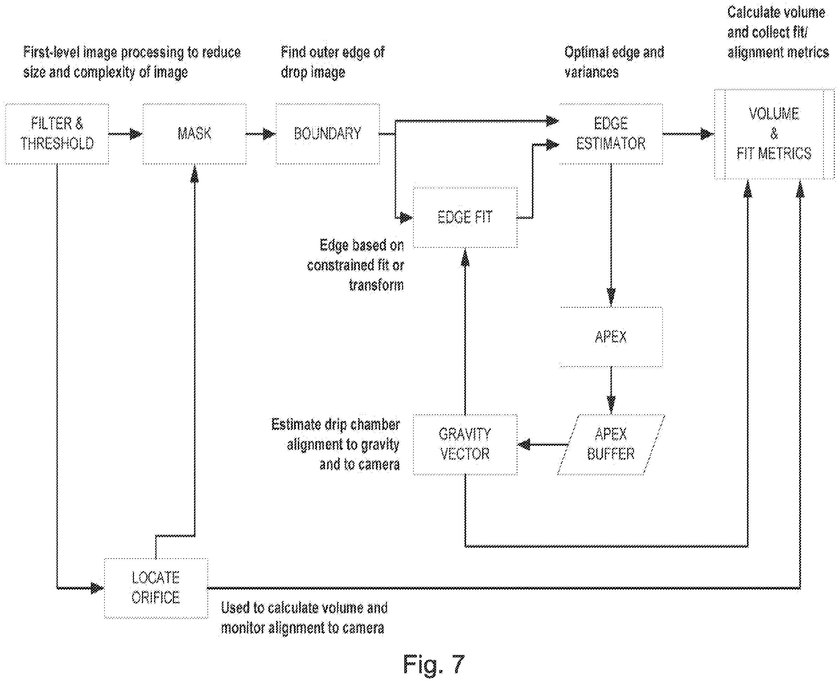

Figure 7 is a flow chart illustrating operation of pump **100** with an optical imaging system. Figure 7 illustrates an example algorithm usable by pump **100**. It should be understood that other algorithms are usable by the pump. The image of drop **124** is filtered and thresholded to create a binary image. Filter operations can include median filtering (to remove isolated glare), background and image uniformity correction (to remove noise sources due to dark noise, read noise, pixel non-uniformity, and illumination non-uniformity), and edge definition (using techniques such as convolution or unsharp masking). The resulting images are thresholded to yield binary images. A binary image consists of values that are either black or white, with no intermediate grayscale values. The images are also processed (in parallel with the above operations) to find the reference location, for example, end point **114**, using techniques such as feature detection, pattern matching, or transform techniques such as the Radon transform. The end point location is used to form an image mask. A mask isolates a region of an image for further processing. Use of a mask increases computational speed, as well as eliminates artifact information from being further processed.

In one embodiment, the binarized, masked images are then processed row-by-row to find the extreme right- and left-boundaries. This boundary-constrained fit is one estimate of the drop edge shape. In one embodiment, the images are also processed using a fit-constrained algorithm. Such an algorithm applies constraints based on assumptions about the drop shape as discussed supra and infra. The constraints are used in a non-linear least squares optimization scheme to minimize the error between the parameterized constraint function(s) and the set of binarized edge images.

The two different edge approximations are provided to an Edge Estimator algorithm that compares fit-constrained images to boundary-constrained images. In the simplest instantiation, the images are compared row-by-row. The boundary-constrained images are considered to be the "correct" result unless they deviates from the fit-constrained images by more than a certain parameter (this parameter is adjusted during calibration). If the deviation is too large, the value from the fit-constrained image is used to replace that of the boundary-constrained image for that row. The above is intended to illustrate the concept behind the estimator. In actual use, more sophisticated algorithms are used to simultaneously optimize the difference between the two initial estimates. An example of such an algorithm is a Kalman filter, but other algorithms familiar to those skilled in the art may also be utilized.

The output from the Edge Estimator also provides the location of the apex of the drop, which is for example, used to calculate the time-dependent gravity vector. This operation requires access to prior estimates of the apex value (to calculate the change), and hence a number of prior values are stored in a buffer. The gravity vector is required for some of the parametric fit functions that are used in the fit-constrained edge estimation algorithms. Hence, the gravity vector is used in a feedback loop for the edge fit algorithms.

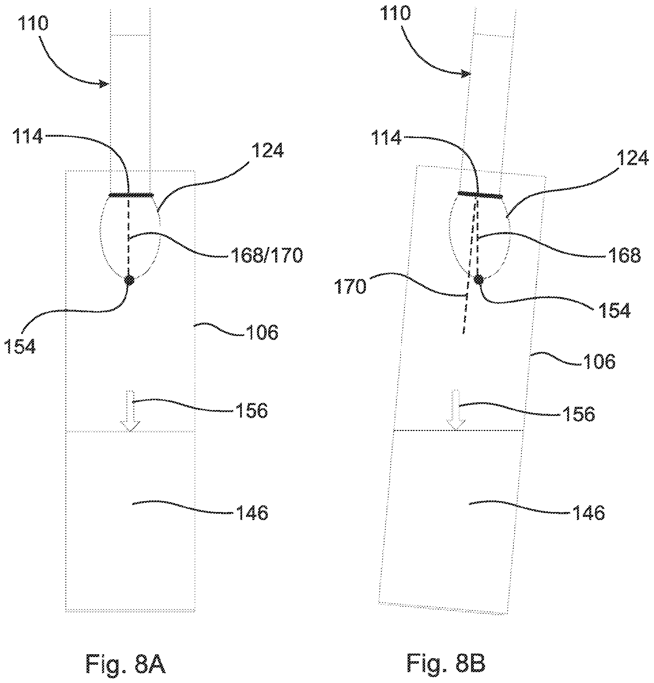

Figures 8A and 8B are schematic details for pump **100** implementing an operation for determining gravity vector **156**. In one embodiment, system **118** illuminates end point **114** and drop **124** and the optical system, for example, sensor **126**, receives light emanating from the end point and light emanating from the drop and transmits data **129** regarding the received light. The microprocessor generates, using the data, respective images of the drop and the end of the drip tube and locates an apex of the drop, the apex being a portion of the drop at a furthest distance from the end of the drip tube. The microprocessor determines, using the location of the apex, an orientation of the drop with respect to the end of the drip tube and calculates, using the orientation of the drop with respect to the end of the drip tube, an orientation of the drip chamber. In one embodiment, the microprocessor compares the orientation of the drip chamber to a set point, for example, a certain orientation with respect to plumb stored in the microprocessor, and generates an out of bound condition alarm when the orientation equals the set point or varies from the set point by a specified amount. For example, if the drip chamber is too far out of plumb, operation of pump **100** may be compromised and the alarm is generated.

For example, in Figure 8A line **168** for the actual orientation of the drop and axis **170** for the drip chamber are co-linear, Since the drop must necessarily align with the forces of gravity (is plumb), the drip chamber is in a plumb orientation in Figure 8A. Also, line **168** is aligned with gravity vector **156**. In Figure 8B, lines **168** and **170** are not co-linear and the drip chamber is not plumb. Thus, in one embodiment, the microprocessor generates lines **168** and **170** and compares the respective locations or orientation of the lines. That is, the microprocessor calculates the orientation of the drip chamber with respect to the gravity vector. In one embodiment, when data **129** is used to generate respective images over a period of time (temporally sequential images), the gravity vector is determined by measuring in the images of the end of the drip tube and the drop, the location of the apex of the pendant drop as it grows over time and tracking the time-dependent directional change of the apexes over a series of these measurements. In one embodiment, the boundary of end **114** is calculated as described supra and the boundary is used as reference plane for calculating the orientation of the drop and/or the drip chamber.

In one embodiment, the illumination system controls illumination properties of the light illuminating the end of the drip tube and the drop and the microprocessor: identifies respective boundaries of the end of the drip tube and the drop from the respective images; fits a parametric function to the respective boundaries; and integrating the parametric function to obtain a volume of the drop, for example, as described above.

In one embodiment, the end point location, gravity vector, and optimal edge estimate are input to a volume calculation routine that integrates the edge image using the "circular disk" assumption discussed above. The location of the end of the drip tube is used to determine the upper limit of integration, while the gravity vector is used to determine the direction of the horizontal (at right angles to the gravity vector). These end and gravity data values are provided along with the volume as output from the algorithm. In one embodiment, the algorithm also passes out the parameters of the edge fit, as well as statistical data such as fit variances. In one embodiment, the preceding information is used in the digital signal processing chain discussed below.

A number of methods can be used to fit a constraint to the measured image. In one embodiment, a "pendant drop" approach, involves solving the Laplace-Young equation (LYE) for surface tension. A drop hanging from a contact point (the end point) has a shape that is controlled by the balance of surface tension (related to viscosity) and gravity. The assumption is only strictly valid when the drop is in equilibrium; oscillations (due to vibration or pressure fluctuations) will distort the drop shape from the Laplace-Young prediction. However, small oscillations will not cause the fit to fail; in fact, the deviation from a fit is itself a good indicator of the presence of such oscillations.

In one embodiment, a Circular Hough Transform (CHT) is used on the image to identify the component of the image that represents the curved bottom of the drop. While not strictly a "fit", the CHT provides a parametric representation of the drop that is characterized by the value and origin of the radius of a circle. The CHT algorithm is representative of a constraint that is determined or applied in a mathematical transform space of the image. Other widely-used transforms, familiar to those skilled in the art, are the Fourier and wavelet transforms, as well as the Radon transform.

The parametric fitting procedures described above apply strong constraints on the possible location of the edge of the drop. Along with the assumption of continuity (a fluid edge cannot deviate from its neighbors over sufficiently short distances), and the requirement that the drop edge terminate at the drip tube orifice, the procedures are used to augment and correct the boundary-constrained image, as discussed above. Other fitting procedures work similarly to those discussed herein.

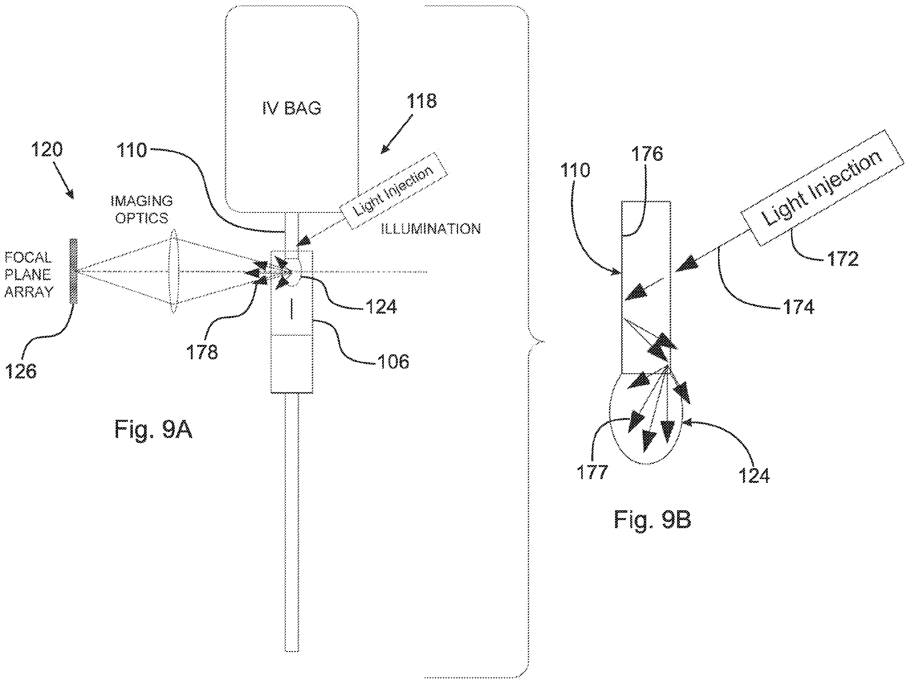

Figures 9A and 9B are schematic details of pump **100** using light injection. Drip tube **110**, drip chamber **106**, tube **108**, drop **124**, imaging system **120**, and sensor **126** are as described for Figure 2. Illumination system **118** includes illumination source **172** for transmitting, or injecting, light **174** into the drip tube. The light reflects off a plurality of portions of internally facing surface **176** of the drip tube and the reflected light is transmitted through the end point **114** of the drip tube into interior **177** of drop **124** such that the interior is uniformly illuminated. The optical system receives light **178** transmitted from the interior of the drop and transmits, to the computer processor, data regarding the received light. The data regarding the received light can be operated upon using any of the operations noted supra For example, in one embodiment, the illumination system is for controlling illumination properties of the light transmitted to the drop, and the optical system is for receiving light from the drop. The microprocessor is for: generating an image from the data, the image including a boundary of the drop; fitting a parametric function to the boundary of the drop; and integrating the parametric function to obtain a volume of the drop.

Thus, light **174** is formed into a beam, which is injected into the transparent drip tube so as to undergo significant internal reflection (i.e., equal to or greater than the so-called "critical angle"). The cylindrical bore of the tube causes the internal reflections to diverge inside the tube (filling the bore of the tube), while imperfections in the tube surface introduce light scattering. The result is that the drop is illuminated internally. Under these conditions the imaging optics in system **120** receive only light that is scattered from the drop surface (there is no direct ray path for the light to reach the lens). In addition to a high contrast edge image, this approach enables the use of a very compact illumination element.

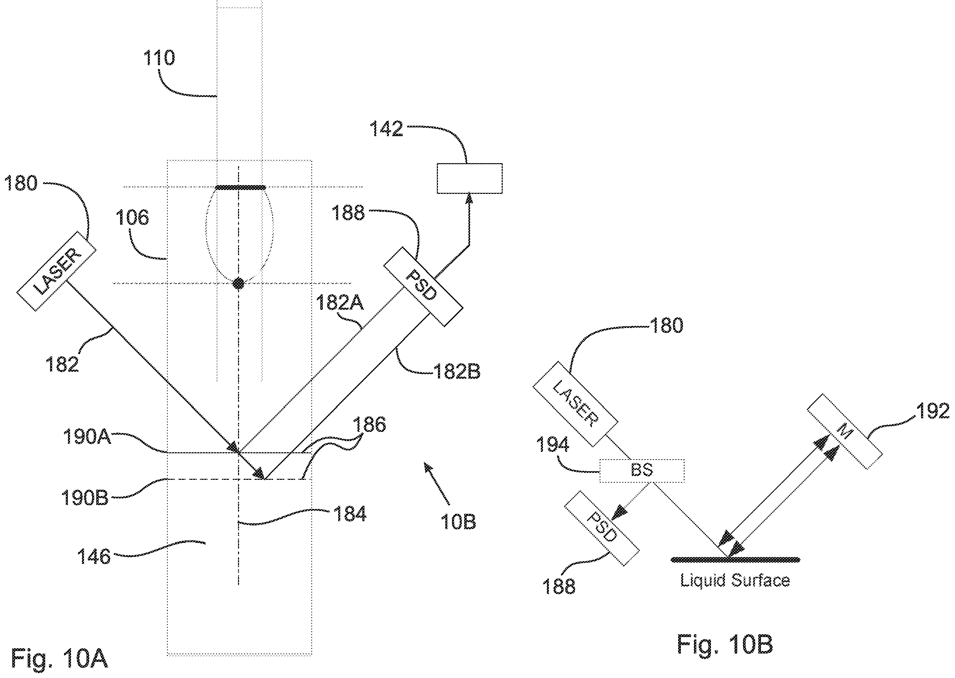

Figure 10A is a schematic detail of pump **100** with a meniscus detection arrangement. Drip tube **110**, drip chamber **106**, tube **108**, and fluid **146** are as described for Figure 2. Imaging system **102** includes light source, for example, a laser, for transmitting light **182** at an acute angle with respect to longitudinal axis **184** for the drip chamber, into the drip chamber such that the light reflects, at the acute angle, off a surface **186** of fluid pooled within the drip chamber. System **102** also includes sensor, or position sensitive detector, **188** for receiving reflected light **182** and transmitting, to the computer processor, data regarding the received light. The microprocessor is for calculating a position of surface **186** using the data regarding the received light.

The location on sensor **188** receiving light **182** depends on the location of surface **186**. Levels **190A** and **190B** show two possible levels for fluid **146** and hence, two possible locations for surface **186**. As seen in Figure 10B, light **182A** and **182B** reflecting from levels **190A** and **190B**, respectively, strike different portions of sensor **188**. The microprocessor uses the difference between the locations on sensor **188** to determine the level of fluid **146**, that is, the meniscus, in the drip chamber. Sensor **188** can be any positional sensitive detector known in the art, for example, a segmented sensor or a lateral sensor. In one embodiment, the microprocessor generates an empty bag alarm or an air-in-line alarm for an instance in which the light transmitted from light source **188** is not received by the optical system, for example, the drip chamber is empty or level **186** is so low that light **182** does not strike fluid **146**.

A segmented positional sensitive detector includes multiple active areas, for example, four active areas, or quadrants, separated by a small gap or dead region. When a symmetrical light spot is equally incident on all the quadrant, the device generates four equal currents and the spot is said to be located on the device's electrical center. As the spot translates across the active area, the current output for each segment can be used to calculate the position of the spot. A lateral positional sensitive detector includes a single active element in which the photodiode surface resistance is used to determine position. Accurate position information is obtained independent of the light spot intensity profile, symmetry or size. The device response is uniform across the detector aperture, with no dead space.

Figure 10B is a schematic detail of pump **100** with a meniscus detection arrangement. In one embodiment, imaging system **102** includes mirror **192** on the opposite side of the drip tube to reflect light **182** back through the drip tube and beam splitter **194** to direct the reflected light to sensor **188**. This configuration enables placement of all the electronics for the optical components on the same side of the tube.

The following provides further detail regarding meniscus level measurement. The drip chamber remains partially filled with fluid at all times during operation. The air trapped in the drip chamber is in pressure equilibrium with the fluid above and below it. The difference in pressure across the air gap drives fluid out of the bottom of the drip chamber and through downstream tubing **108**. Fluid enters and leaves the drip tube chamber continuously as the drop grows in volume, and hence the meniscus level of the fluid remains nearly constant. However, changes in the meniscus level can occur for several reasons: transient changes may occur when a drop detaches and falls into the fluid below; or fluctuations may occur due to pressure oscillations in the fluid (due to pump vibration, motion of the tubing set, or motion of the patient). These transient changes will fluctuate around a mean meniscus value, and hence do not indicate changes in flow rate over times long compared to the characteristic fluctuation times.

Variations that change the mean meniscus level over longer times may occur due to changes in the external pressure environment (e.g., in a traveling vehicle or aircraft), changes in backpressure arising from medical issues with the patient, or due to occlusions or other malfunctions in the pumping process. These long-term meniscus level changes represent a concomitant change in the overall flow rate, and may be used to provide a refinement to the flow measurements described supra Hence, it may be desired to monitor the level of the meniscus during the infusion, and to use the information derived therein as an indicator of operational problems with the infusion system, or as an adjunct to the primary optical flow measurement.

The method described above for measuring the level of fluid **146** uses the reflection of a light beam from the top surface of the fluid in the drip chamber. The axis of the reflected beam is shifted (deflected) laterally as the fluid level changes, for example, as shown by light **182A** and **182B**. The amount of deflection depends only on the fluid level change, and on the incident angle of the beam. Although a laser light source is shown in the figure, the technique is compatible with any light beam. Further, although the beam is shown freely propagating, the system may also incorporate lens elements to control the beam.

In one embodiment (not shown), sensor **126** (the imaging focal plane array) is used both for imaging drop **124** and measuring the meniscus of fluid **146** via beam splitters and other simple optics. Sensor **126** can be shared in at least two ways: a portion of the sensor that is not used for pendant drop imaging can simultaneously record the deflected beam; or illumination system **118** for pendant drop imaging and meniscus level measurement can be alternated in time, such that the sensor alternately records the drop image and the deflected beam image. For example, pump **100** can combine the imaging systems **102** shown in Figures 2 and 10A/10B or shown in Figures 2 and 9A.

Thus, in one embodiment, system **102** includes a first light source, such as light source **172** for transmitting light into the drip tube such that the light reflects off an internally facing surface of the drip tube, and the reflected light is transmitted through the end of the drip tube into an interior of a drop of the IV fluid hanging from the first end of the drip tube. System **102** also includes a second light source, such as light source **188**, transmitting light, at an acute angle with respect to a longitudinal axis for the drip chamber, into the drip chamber such that the light reflects, at the acute angle, off a surface for IV fluid disposed within the drip chamber. Optical sensor **126** is for: receiving the reflected light transmitted from the interior of the drop; receiving the reflected light from the second light source; and transmitting, to the computer processor, data regarding the received light from the first and second light sources. The microprocessor is for calculating a volume of the drop using the data regarding the light received from the first light source, and calculating a position of the surface of the using the data regarding the light received from the second light source, as described supra.

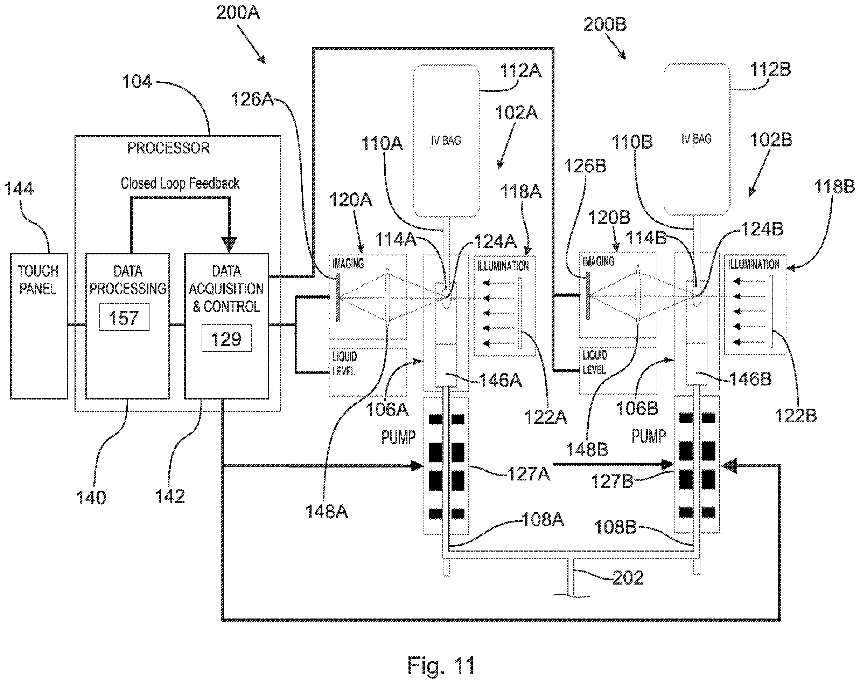

Figure 11 is a schematic block representation of pump assemblies **200A** and **200B** with respective optical imaging system in a primary and secondary configuration. The assemblies include the components for pump **100** described supra, with the exception of the processor and control panel. In general, the description above regarding the operation of pump **100** is applicable to the operation of assemblies **200A** and **200B**. Assembly **200A** is connected to primary fluid source **112A**. Pump **200B** is connected to primary fluid source **112B**. Sources **112A** and **112B** are arranged in a primary/secondary infusion configuration. For example, a primary medication in source **112A** is administrated in coordination with a secondary medication in source **112B**. As is known in the art, in a primary/secondary configuration, the medication in the secondary source is infused before the medication in the primary source. Tubings **108A** and **108B** from pump mechanisms **127A** and **127B**, respectively, are connected to common tubing **202**.

In one embodiment, a single processor and control panel, for example, processor **104** and panel **144** are used for assemblies **200A** and **200B.** The processor operates assembly **200B** according to appropriate protocols until the regime for the fluid in source **112B** is completed. Then, the processor automatically deactivates assembly **200B** as required and begins the infusion of the fluid in source **112A**. In one embodiment (not shown), each assembly has a separate processor and control panel or each assembly has a separate processor and a common control panel.

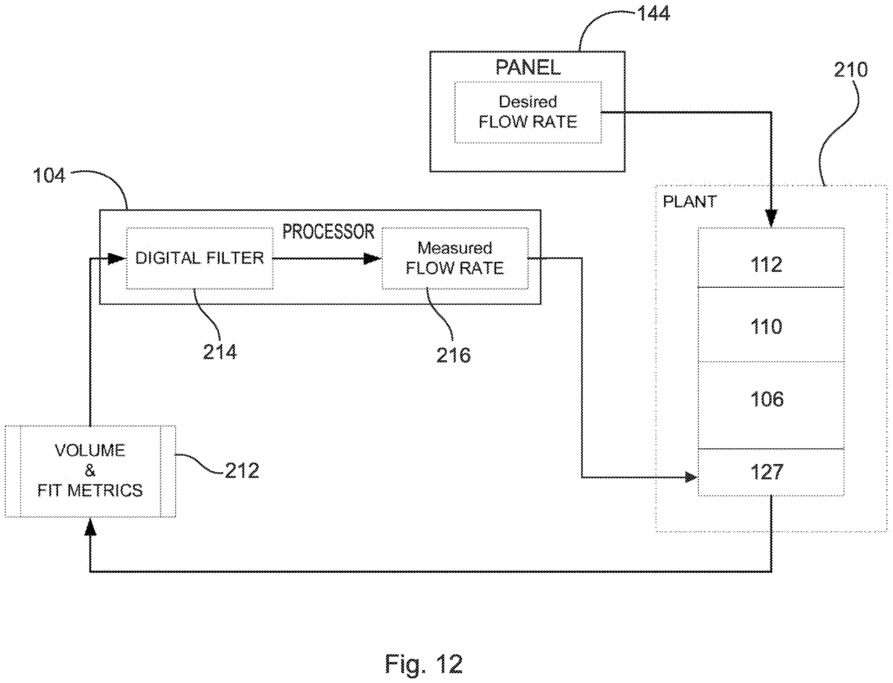

Figure 12 is a top-level block diagram illustrating operation of pump **100** with an optical imaging system. In one embodiment, the volume measurement, and fit metrics if applicable, described above are fed into a digital signal processing algorithm that calculates the flow rate and provides feedback to the pump control system. Plant **210** includes source **112**, the drip chamber, the drip tube, and pump mechanism **127**. The microprocessor outputs the Volume and Fit Metrics **212**, which are filtered by digital filter **214** in a portion of the microprocessor to provide measured flow rate **216**. The measured flow rate is compared with the desired flow rate, for example, input into the microprocessor via panel **144**, closing the feedback loop for pump **100**.

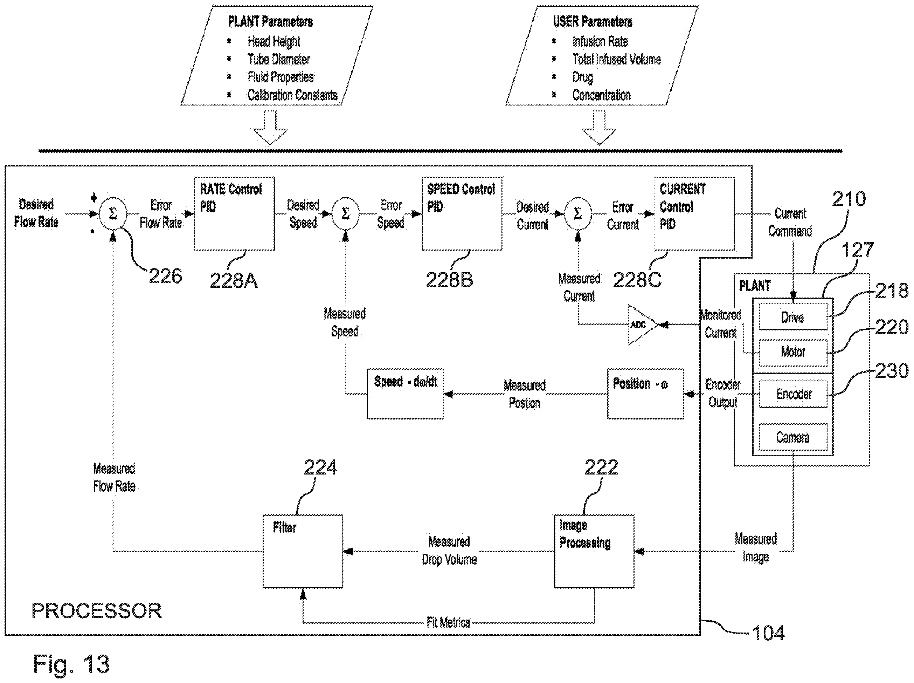

Figure 13 is a block diagram illustrating example signal processing and feedback control for pump **100** with an optical imaging system. Mechanism **127** includes drive **218** and motor **220**. Imaging data from system **102** is processed by image processing block **222** to generate a Measured Drop Volume, and the results are input to filter block **224**. The output of the filter block is the Measured Flow Rate. The Measured Flow Rate is compared to the Desired Flow Rate by comparator **226**, providing the Error Flow Rate (error estimate). The Error Flow Rate feeds into a staged series of PID (Proportional, Integral, Derivative) control algorithms **228**. Each PID block operates on a successively faster time scale. Block **228A** controls the flow rate, block **228B** controls the pump motor speed, and block **228C** controls the pump motor current. The speed control incorporates feedback from motor position encoder **230.** The current control incorporates feedback from a motor current sensor in motor **220**.

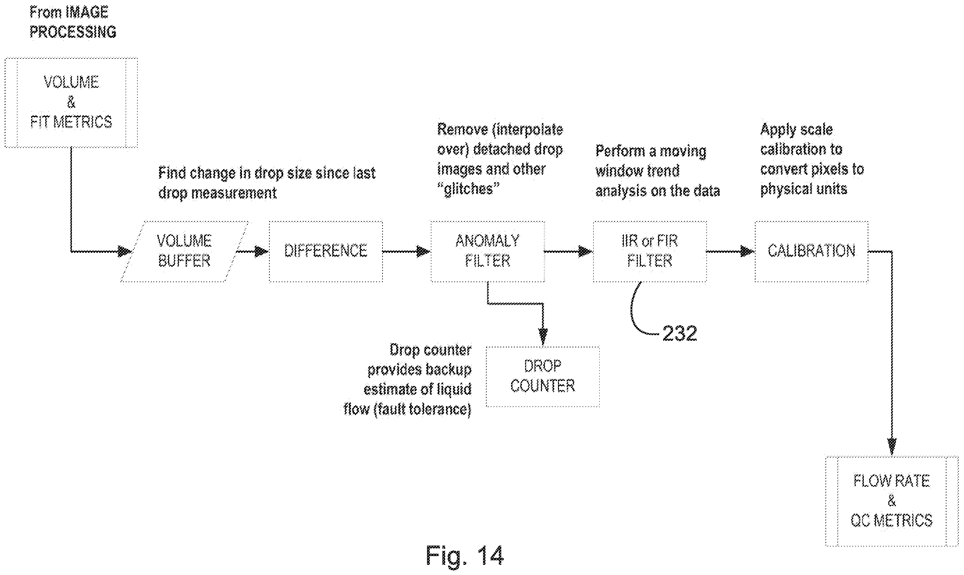

Figure 14 is a block diagram illustrating example digital filtering in pump **100** with an optical imaging system. Filter **232** can be any filter known in the art, for example, the general class of FIR/IIR filters known to those skilled in the art. A simple example is an FIR filter that implements a time average over a number of samples.

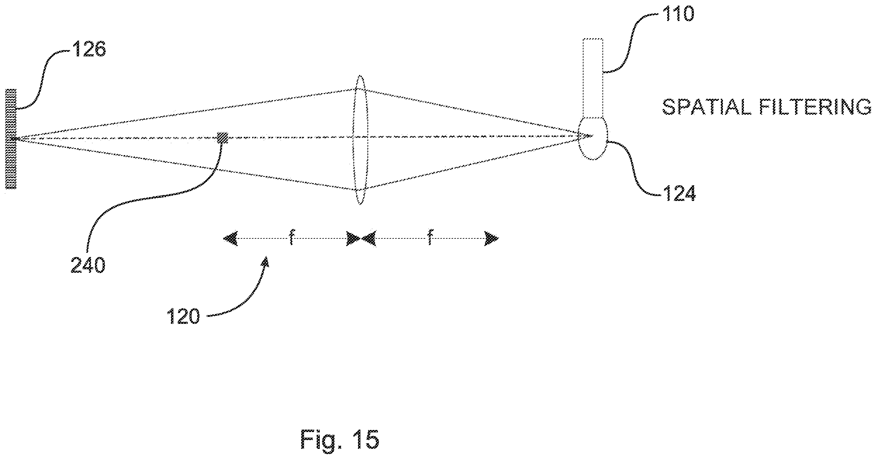

Figure 15 is a schematic representation of example spatial filtering in pump **100** with an optical imaging system. The goal of high resolution and edge definition for images of drop **124** are attained by illumination techniques, optical techniques, or both, for example, as described supra. In one embodiment, spatial filtering techniques are used in the optics for system **120**. For example, mask **240** at the back focal plane of imaging system **102** modifies (via optical Fourier transform) the image generated by the optical system, for example, sensor **126**. A DC block filter is shown in Figure 15. This filter blocks the central cone of the transmitted light and enhances edge images (associated with scattered light).

In one embodiment, the sensitivity of sensor **126** is matched to the illumination spectrum of the light source in system **118**. In one embodiment, sensor **126** is a low-cost visible light sensor (400-1000 nm wavelength) and source **122** generates light that is outside the range of human visual perception (i.e., 800-1000 nm). In this case the operator will not be distracted by the bright illumination source.

It should be understood that pump **100** can be any pump mechanism or pump application known in the art and is not limited to only IV infusion pump applications. In the case of a gravity-fed system, the pumping mechanism can be replaced by a valve or flow restrictor, and still be compatible with the configurations and operations described supra.

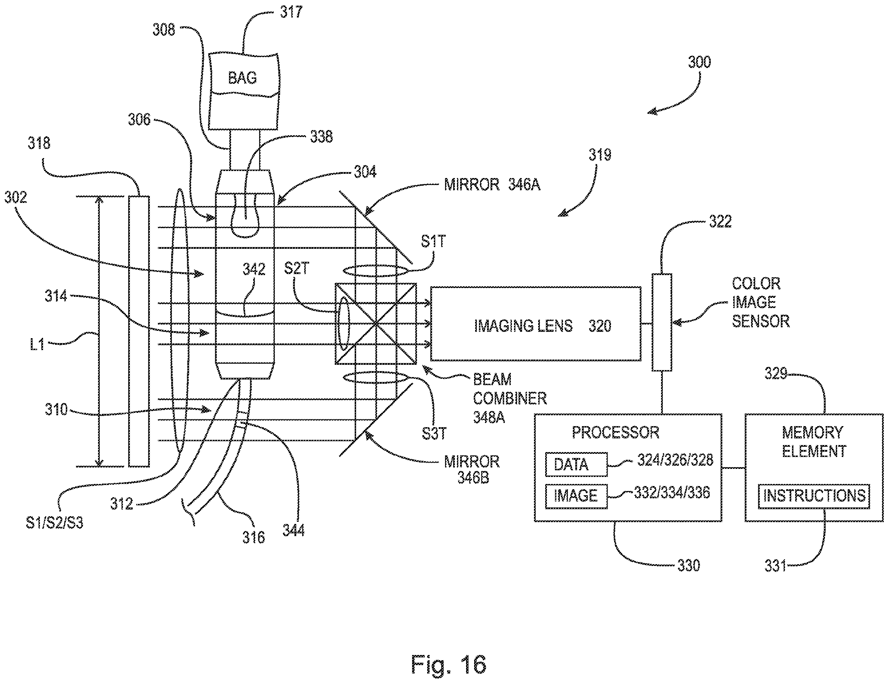

Figure 16 is a schematic representation of optical imaging system **300** with multiple imaging channel optical sensing. In an example embodiment, system **300** is used with infusion tube **302** including drip chamber **304**. Drip chamber **304** includes portion **306** with drip tube **308**, portion **310** including exit port **312**, and portion **314** between portions **306** and **310.** Output tube **316** can be connected to exit port **312** for flowing fluid out of drip chamber **304**. Drip tube **308** is for connection to source of fluid **317**, for example, medication bag **317**. System **300** includes at least one light source **318** for emitting spectrums **S1**, **S2**, and **S3** of light, and optical system **319**.

Light source **318** can be any light source known in the art, including, but not limited to a light-emitting diode (LED), an array of LEDs, a laser diode, an incandescent lamp, or a fluorescent lamp.

The optical system includes single lens **320** for receiving and transmitting **S1T**, **S2T**, and **S3T**. **S1T**, **S2T**, and **S3T** include spectrums **S1**, **S2**, and **S3**, transmitted through portions **306**, **310**, and **314**, respectively. Optics system **319** includes single image sensor **322** for receiving **S1T**, **S2T,** and **S3T** from single lens **320.** Sensor **322** generates and transmits data **324**, **326**, and **328**, characterizing **S1T**, **S2T**, and **S3T**, respectively, received by lens **320.** System **300** includes memory element **329** and at least one specially programmed processor **330.** Memory element **329** is configured to store computer executable instructions **331**. Processor **330** is configured to execute instructions **331** to generate, using data **324**, **326**, and **328,** images **332**, **334**, and **336** of portions **306**, **310**, and **314**, respectively.

By "characterize" we mean that the respective data describes, or quantifies, the spectrum of light, for example, providing parameters enabling generation of an image using the respective data. By "emitting light" we mean that the element in questions generates the light. By "transmitted by" we mean passing light through the element in question, for example, light emitted by light source **318** passes through portions **306**, **310**, and **314**.

In an example embodiment, sensor **322** is a color image sensor. In an example embodiment, light source **318** is a single light source.

In an example embodiment, portion **306** includes drop **338** pendant from drip tube **308** and image **332** includes an image of drop **338**. Processor **330** is configured to execute instructions **331** to determine a volume of pendant drop **338** using image **332**. The volume can be used in control schemes to regulate flow of fluid through infusion tube **302**.

In an example embodiment, portion **314** includes meniscus **342** for fluid in drip chamber **304** and image **336** includes an image of meniscus **342**. Processor **330** is configured to execute instructions **331** to determine a position of meniscus **342** using image **336**. The position can be used in control and alarm schemes to regulate flow of fluid through infusion tube **302.** In an example embodiment, air bubble **344** is present in portion **310** and processor **330** is configured to execute instructions **331** to determine a volume of air bubble **344** using image **334**. The volume can be used in alarm schemes to ensure safe operation of infusion tube **302**.

In an example embodiment, light source **318** emits red, blue, and green spectrum light. In an example embodiment, **S1T** consists of one of the red, blue, or green spectrum light, **S2T** consists of one of the red, blue, or green spectrum light not included in **S1T**, and **S3T** consists of one of the red, blue, or green spectrums of light not included in **S1T** or **S2T**. Thus each of **S1T**, **S2T**, and **S3T** consists of one of red, blue, or green light not included in the other of **S1T**, **S2T**, and **S3T**. That is, each of **S1T**, **S2T**, and **S3T** is different from the others. By "red spectrum light" we mean light including wavelengths between about 610 nm and 675 nm, with peak intensity at about 625 nm. By "blue spectrum light" we mean light including wavelengths between about 410 nm and 480 nm, with peak intensity at about 470 nm. By "green spectrum light" we mean light including wavelengths between about 500 nm and 575 nm, with peak intensity at about 525 nm. Thus, the respective spectrums for red, blue, and green light do not have overlapping wavelengths.

In an example embodiment, system **300** includes mirror **346** for reflecting one only of **S1T**, **S2T**, and **S3T**. For example, mirror **346A** reflects **S1T**. In an example embodiment, system **300** includes mirror **346A** for reflecting one only of **S1T**, **S2T**, or **S3T**, and mirror **346B** for reflecting another only of **S1T**, **S2T**, or **S3T**, for example, **S3T**. In an example embodiment, system **300** includes beam combiner **348A** for reflecting two only of **S1T**, **S2T**, or **S3T**. For example, in Figure 16, beam combiner **348A** reflects **S1T** and **S3T** and transmits **S2T**.

The following provides further detail regarding Figure 16. As described below, various filtering operations are used to generate **S1T**, **S2T**, and **S3T**. Mirror **346A** receives the combined red, blue, and green spectrums emitted by source **318** and transmitted by portion **306** of drip chamber **304**, but reflects only spectrum **S1T**. Mirror **346B** receives the combined red, blue, and green spectrums emitted by source **318** and transmitted by portion **310** of output tube **316**, but reflects only spectrum **S3T**. Thus, mirror **348A** and **348B** are color-filtering.

In an example embodiment, sensor **322** is not monochrome, that is, sensor **322** is a color image sensor. Beam combiner **348A** transmits only spectrum **S2T** emitted by source **318** and transmitted by portion **314** of drip chamber **304**. Specifically, beam combiner **348A** receives the combined red, blue, and green spectrums emitted by source **318** and transmitted by portion **314** of drip chamber **304**, but only transmits spectrum **S2T**. The beam combiner also reflects spectrum **S1T** reflected by mirror **346A** and spectrum **S3T** reflected by mirror **346B**. Note that the reflecting operations of beam combiner **348A** can be implemented using broad-band reflection, since mirrors **346A** and **346B** have filtered out spectrums **S2T** and **S3T** and spectrums **S1T** and **S2T**, respectively.

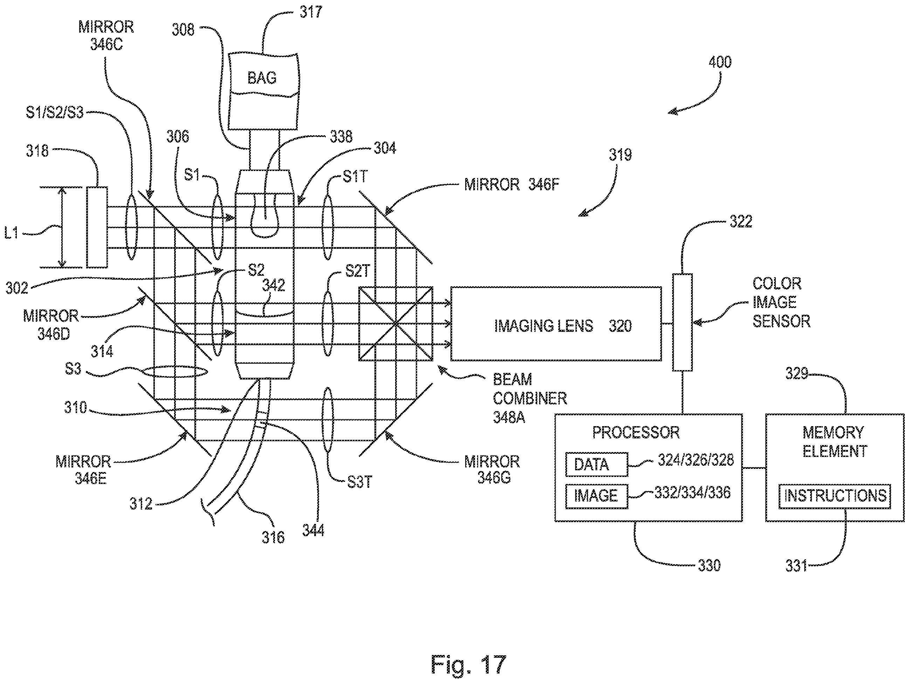

Figure 17 is a schematic representation of optical imaging system **400** with multiple imaging channel optical sensing. The discussion regarding system **300** is applicable to pump **400** except as follows. In an example embodiment: optics system **319** includes a mirror for transmitting to one of portions **306**, **310**, or **314** one only of **S1**, **S2**, or **S3**; or optics system **319** includes a mirror for reflecting to one of portions **306**, **310**, or **314**, one only of **S1**, **S2**, or **S3**. For example: mirror **346C** transmits **S1** to portion **306** and reflects **S2** and **S3**; mirror **346D** transmits **S3** and reflects **S2** to portion **314**; and mirror **346E** reflects **S3** to portion **310**. In an example embodiment, mirror **346E** is a broad-band reflecting mirror.

Mirror **346F** is for reflecting spectrum **S1T** transmitted by portion **306** of drip chamber **304** to beam combiner **348A**. In an example embodiment, mirror **346F** is a broad-band reflecting mirror. Mirror **346G** is for reflecting spectrum **S3T** transmitted by portion **310** of drip chamber **304** to beam combiner **348A**. In an example embodiment, mirror **346G** is a broad-band reflecting mirror. Since the light entering beam combiner **348A** has been separated into discrete spectrums, for example, light from mirror **346G** is only spectrum **S2T**, broad-band transmitting and reflecting operations can be used in beam combiner **348A**.

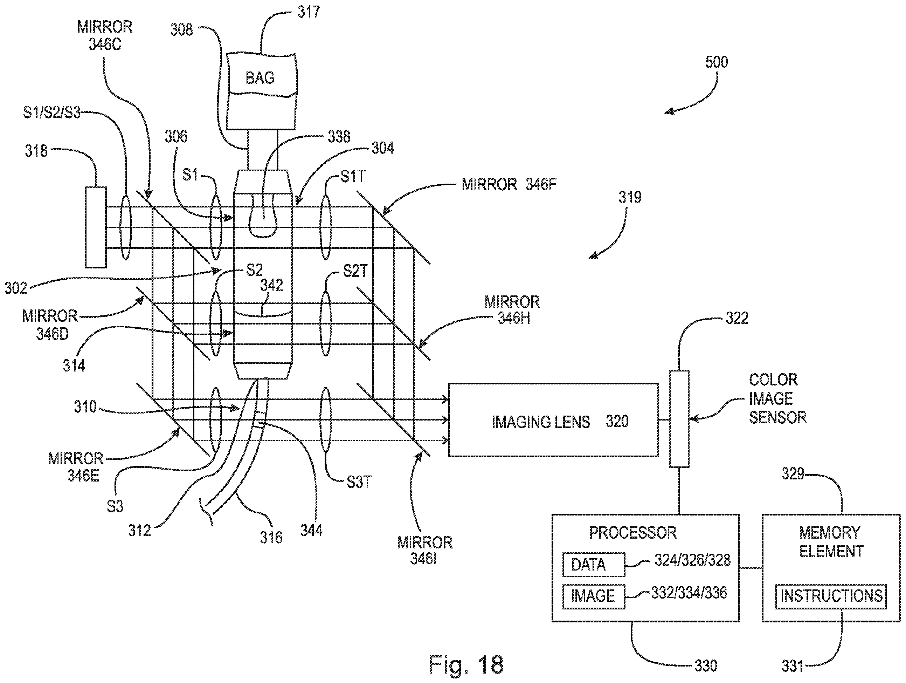

Figure 18 is a schematic representation of optical imaging system **500** with multiple imaging channel optical sensing. The respective discussions regarding systems **300** and **400** are applicable to system **500** except as follows. In an example embodiment, optical system **319** replaces a beam combiner with mirrors **346H** and **346I.** Mirror **346H** transmits **S1T** reflected by mirror **346F** and reflects **S2T** (from mirror **346D**). Mirror **346I** transmits **S3T** (from mirror **346E**) and reflects **S1T** (transmitted by mirror **346H**) and **S2T** (reflected by mirror **346H**).

In Figure 16, length **L1** of light source **318** must be sufficient to span portions **306**, **310**, and **314**, since light source **318** must emit light directly through portions **306**, **310**, and **314**. However, in Figures 17 and 18 length **L1** of light source **318** is considerably less, for example, equal only to length **L2** of portion **306**. In Figures 17 and 18, light source **318** is emitting light directly through portion **306**; however, combinations of mirrors are used to reflect light to portions **310** and **314**. Thus, a smaller and less expensive device can be used for light source **318** in Figures 17 and 18.

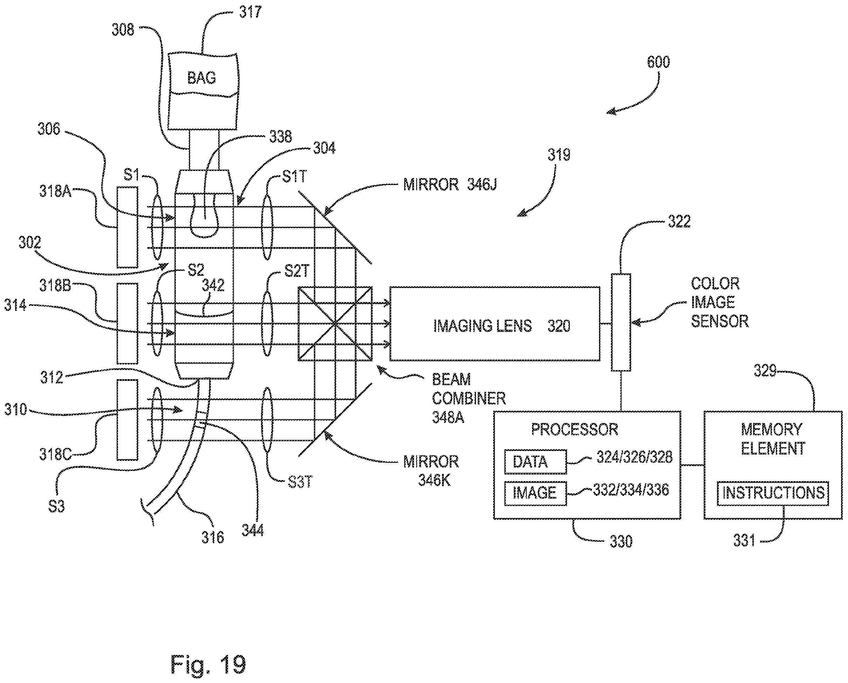

Figure 19 is a schematic representation of optical imaging system **600** with multiple imaging channel optical sensing. The discussion regarding system **300** is applicable to system **600** except as follows. System **600** includes three light sources: light source **318A** for emitting only spectrum **S1**, light source **318B** for emitting only spectrum **S2**, and light source **318C** for emitting only spectrum **S3**. Optical system **319** includes mirror **346J** for reflecting **S1T** and mirror **346K** for reflecting **S3T**. Beam combiner **348B** transmits **S2T** and reflects **S1T** and **S3T**. In an example embodiment, one or both of mirrors **346J** and **346K** are broad-band reflecting mirrors. In an example embodiment, beam combiner **348B** has broad-band transmitting and reflecting functionality.

In respective example embodiments for system **300**, **400**, **500**, and **600**, two-channel imaging is performed for only two of portions **306**, **310**, or **314** and imaging is not performed on the remaining portion **306**, **310**, or **314**.

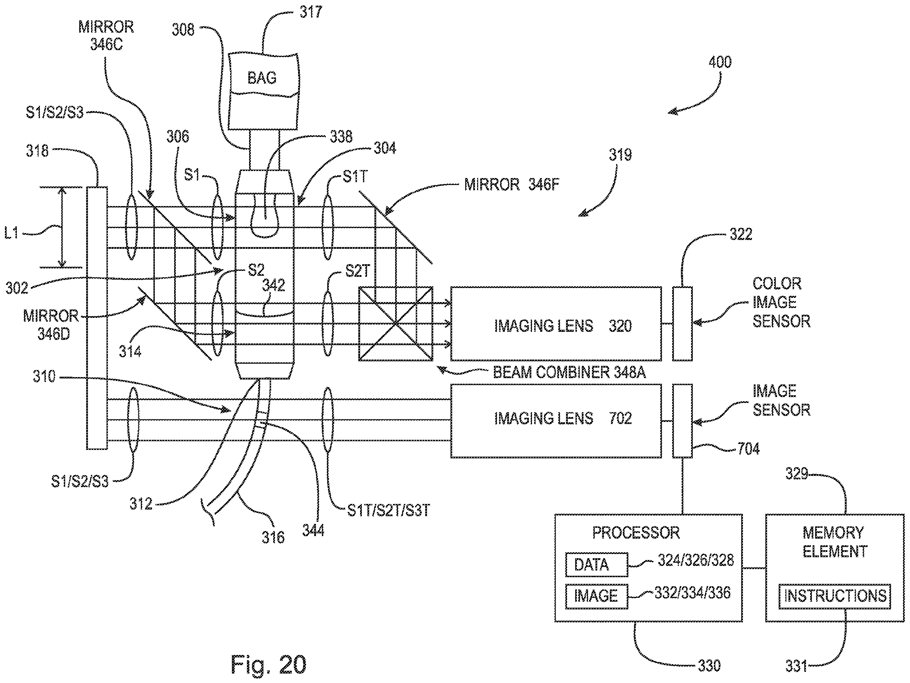

Figure 20 is a schematic representation of optical imaging system **700** with two-channel optical imaging and a single light source. In system **700**, chromatic multiplexing is implemented for only two of portions **306**, **310**, or **314**. System **700** can use system **400** as a starting point. The following describes differences between systems **400** and **700** as shown. In Figure 20, two-channel optical imaging is implemented for portions **306** and **314**. Mirrors **346E** and **346G** are removed. Mirror **346D** no longer is required to transmit **S3**. Beam combiner **348A** is no longer required to reflect **S3T**. Otherwise, the operations regarding portions **306** and **314** are the same as described for Figure 17. In an example embodiment, imaging of portion **310** is implemented by adding lens **702** to receive light **S1T/S2T/S3T** transmitted through portion **310** from light source **318**. Lens **702** transmits **S1T/S2T/S3T** to image sensor **704**, which generates data **326**. Processor **330** generates image **334** from data **326**. Image sensor **704** can be monochromatic, since chromatic multiplexing is not being implemented for portion **310**.

Other combinations of two-channel optical sensing are possible for system **700** as is apparent to one skilled in the art. For example, mirror **346D** can be removed such that two-channel optical sensing is performed for portions **306** and **310** only. Operations as described for portions **306** and **310** for Figure 17 are substantially the same. Lens **702** receives **S1T/S2T/S3T** transmitted by portion **314** and transmits **S1T/S2T/S3T** to image sensor **704**, which generates data **328**. Processor **330** generates image **336** from data **328**. Image sensor **704** can be monochromatic. For example, mirror **346F** can be removed such that two-channel optical sensing is performed for portions **310** and **314** only. Operations as described for portions **310** and **314** for Figure 17 are substantially the same. Lens **702** receives **S1T/S2T/S3T** transmitted by portion **306** and transmits **S1T/S2T/S3T** to image sensor **704**, which generates data **324**. Processor **330** generates image **332** from data **324**. Image sensor **704** can be monochromatic. It should be understood that other configurations of components in system **400** are possible to implement two-channel optical imaging. In an example embodiment, two-channel imaging is performed for only two of portions **306**, **310**, or **314** and imaging is not performed on the remaining portion **306**, **310**, or **314**. That is, a second lens and image sensor are not employed to image the remaining portion **306**, **310**, or **314**.

System **300** can be modified for two-channel operation as is apparent to one skilled in the art. For example, two-channel operation can be implemented for portions **306** and **314** only by removing mirror **346B.** Operations as described for portions **306** and **314** for Figure 16 are substantially the same. **S1T/S2T/S3T** from portion **310** is received by a second lens (not shown) and transmitted to a second image sensor (not shown) that can be monochromatic. The second sensor generates data **326** for generating image **334**. For example, two-channel operation can be implemented for portions **310** and **314** only by removing mirror **346A.** Operations as described for portions **310** and **314** for Figure 16 are substantially the same. **S1T/S2T/S3T** from portion **306** is received by a second lens (not shown) and transmitted to a second image sensor (not shown) that can be monochromatic. The second sensor generates data **324** for generating image **332**. For example, two-channel operation can be implemented for portions **306** and **310** only. Operations as described for portions **306** and **310** for Figure 16 are substantially the same. **S1T/S2T/S3T** from portion **314** is received by a second lens (not shown) and transmitted to a second image sensor (not shown) that can be monochromatic. The second sensor generates data **328** for generating image **336**. It should be understood that other configurations of components in system **300** are possible to implement two-channel optical imaging. In an example embodiment, two-channel imaging is performed for only two of portions **306**, **310**, or **314** and imaging is not performed on the remaining portion **306**, **310**, or **314**. That is, a second lens and image sensor are not employed to image the remaining portion **306**, **310**, or **314**.

System **500** can be modified for two-channel operation as is apparent to one skilled in the art. For example, to implement two-channel operation for portions **306** and **314** only, mirror **346E** can removed. Operations as described for portions **306** and **314** for Figure 18 are substantially the same. **S1T/S2T/S3T** from portion **310** is received by a second lens (not shown) and transmitted a second image sensor (not shown) that can be monochromatic. The second sensor generates data **326** for generating image **334.** For example, to implement two-channel operation for portions **310** and **314** only, mirror **346F** can removed. Operations as described for portions **310** and **314** for Figure 18 are substantially the same. **S1T/S2T/S3T** from portion **306** is received by a second lens (not shown) and transmitted a second image sensor (not shown) that can be monochromatic. The second sensor generates data **324** for generating image **332**. For example, to implement two-channel operation for portions **306** and **310** only, mirrors **346D** and **346H** can be removed. Operations as described for portions **306** and **310** for Figure 18 are substantially the same. **S1T/S2T/S3T** from portion **314** is received by a second lens (not shown) and transmitted a second image sensor (not shown) that can be monochromatic. The second sensor generates data **328** for generating image **336**. It should be understood that other configurations of components in system 500 are possible to implement two-channel optical imaging. In an example embodiment, two-channel imaging is performed for only two of portions **306**, **310**, or **314** and imaging is not performed on the remaining portion **306**, **310**, or **314**. That is, a second lens and image sensor are not employed to image the remaining portion **306**, **310**, or **314**.

System **600** can be modified for two-channel operation as is apparent to one skilled in the art. For example, to implement two-channel operation for portions **306** and **314** only, mirror **346K** can removed. Operations as described for portions **306** and **314** for Figure 19 are substantially the same. **S3T** from portion **310** is received by a second lens (not shown) and transmitted a second image sensor (not shown) that can be monochromatic. Light source **318C** can be broadband (emit **S1/S2/S3**). The second sensor generates data **326** for generating image **334.** For example, to implement two-channel operation for portions **310** and **314** only, mirror **346J** can removed. Operations as described for portions **310** and **314** for Figure 19 are substantially the same. **S1T** from portion **306** is received by a second lens (not shown) and transmitted a second image sensor (not shown) that can be monochromatic. Light source **318A** can be broadband (emit **S1/S2/S3**). The second sensor generates data **324** for generating image **332.** For example, to implement two-channel operation for portions **306** and **310** only, **S2T** from portion **314** is received by a second lens (not shown) and transmitted a second image sensor (not shown) that can be monochromatic. Light source **318B** can be broadband (emit **S1/S2/S3**). The second sensor generates data **328** for generating image **336**. Operations as described for portions **306** and **310** for Figure 19 are substantially the same. It should be understood that other configurations of components in system **600** are possible to implement two-channel optical imaging. In an example embodiment, two-channel imaging is performed for only two of portions **306**, **310**, or **314** and imaging is not performed on the remaining portion **306**, **310**, or **314**. That is, a second lens and image sensor are not employed to image the remaining portion **306**, **310**, or **314**.

For the sake of brevity, portions of the following discussion are directed to system 300 in Figure 16; however, it should be understood that the discussion is applicable to Figures 17 through 19 as well. Further, the following discussion is directed to embodiments in which multiple channel optical sensing is implemented for all three of portions **306**, **310**, and **314**. However, it should be understood that the discussion is applicable to the two-channel embodiments discussed above. Using a single lens, such as lens **320**, and image sensor, such as sensor **322**, in place of three lens and sensors, reduces cost and complexity of system **300**. All three spectrums **S1**, **S2**, and **S3**, transmitted by lens **320** are received simultaneously by single image sensor **322**. However, if sensor **322** is a monochrome sensor, conventional signal processing cannot be used to generate images **332**, **334**, and **346**. For example, a monochrome sensor cannot distinguish among the red, blue, and green and cannot use conventional signal processing to separate spectrums **S1T**, **S2T**, and **S3T** to generate images **332**, **334**, and **346**. Advantageously, system **300** uses a color imaging sensor for sensor **322**, which is able to distinguish among spectrums **S1T**, **S2T**, and **S3T**.

Since a single, separate, respective color from the red, blue, and green spectrums is used for each of spectrums **S1T**, **S2T**, and **S3T**, imager **322** is able to transmit data **324**, **326**, and **328** for single respective spectrums and hence, a single respective image of each of portions **306**, **310**, or **314** can be generated using conventional signal processing operations. For example, spectrums **S1T**, **S2T**, and **S3T** can consist of red, blue, and green spectrum light, respectively. The red-responsive pixels of the sensor pick up spectrum **S1T**, the blue-responsive pixels of the sensor pick up spectrum **S2T**, and the green-responsive pixels of the sensor pick up spectrum **S3T**.

Thus, the red-responsive pixels record an image of drop **338**, the blue-responsive pixels record an image of meniscus **342**, and the green-responsive pixels record an image of portion **310**. Thus, each group of responsive pixels (for example, the red-responsive pixels) remain unresponsive to, in essence filtering out, images from the other images corresponding to the remaining groups of responsive pixels (for example, the blue and green-responsive pixels). Thus, there is no overlap of spectrums or images included in data transmitted to processor **330** and conventional signal processing can be used to generate images **332**, **334**, and **346**.

The use of broad-band reflecting mirrors/reflecting operations rather than color filtering reflecting and transmitting can reduce the cost of respective optics systems **319** in Figures 17 through 19.

In an example embodiment (not shown), a single lens, such as lens **320**, and a single monochrome image sensor are used in a time multiplexing arrangement in an infusion pump. For example, using Figure 19 as a reference, each of light sources **318A/B/C** emits the same spectrum of light. The emitted light is transmitted through portions of an infusion tube, such as infusion tube **302**, analogous to portions **306**, **310**, and **314** described supra. Via an arrangement similar to mirrors **346A/346B** and beam combiner **348A**, the light, transmitted through the analogous portions, is transmitted to the single lens, which transmits the light to a processor, such as processor **330**. As noted above, a monochrome sensor cannot distinguish, using conventional signal processing, three simultaneously received images. However, in the example embodiment, the three light sources are sequentially energized such that only one light source is energized per image frame of the image sensor. For example, in a first frame, the light source emitting light transmitted through the portion analogous to portion **306** is energized, in the next frame, the light source emitting light transmitted through the portion analogous to portion **310** is energized, and in the next frame the light source emitting light transmitted through the portion analogous to portion **314** is energized. The processor receives only one image per frame and is able to transmit respective data for each image in each frame to the processor. The processor in turn is able to generate separate images for each of the analogous portions of the pump. The use of a monochrome image sensor and three backlights emitting the same spectrum reduces the cost of the pump in the example embodiment.

The following discussion provides further detail regarding Figures 16 through 19. It should be understood that the following discussion is applicable to the two-channel embodiments discussed above. In an example embodiment, lens elements (not shown) can be added to respective image paths (paths traversed by light from a light source to an image sensor) for systems **300** through **600** to compensate for unequal image paths. In an example embodiment, a spectrum of light in the near infra red range (for example, between 700 nm and 1,000 nm) can be used to illuminate portions **306**, **310**, or **314**. In an example embodiment, light source **318** and/or light sources **318A/B/C** are LEDs and the LEDs are pulsed to improve operating efficiency or create a strobe effect which eliminates motion blur of moving artifacts. The pulsing is synchronized with the shutter speed of image sensor **322**. In an example embodiment, the general configuration of Figure 18, which does not use a beam combiner, is modified by using three light sources as shown in Figure 19. The resulting combination uses fewer mirrors than shown in Figure 18, reducing the cost of the embodiment.

Thus, it is seen that the objects of the invention are efficiently obtained, although changes and modifications to the invention should be readily apparent to those having ordinary skill in the art, without departing from scope of the invention as claimed. Although the invention is described by reference to a specific preferred embodiment, it is clear that variations can be made without departing from the scope of the invention as claimed.

## Claims

1. An optical imaging system (102) for use with an infusion tube having a drip chamber (106) including a first portion with a drip tube (110), a second portion with an exit port (312), and a third portion located between the first and second portions, the optical imaging system being **characterized in that** it further comprises:
at least one light source (122) for emitting at least two of a first, second, or third spectrums of light;
an optics system (319) including:
a single lens (148) for receiving and transmitting at least two of:
the first spectrum of light transmitted through the first portion;
the second spectrum of light transmitted through the second portion; or,
the third spectrum of light transmitted through the third portion;
a single image sensor (322) for:
receiving the at least two of the first, second, or third spectrums of light from the single lens; and,
generating and transmitting data characterizing the at least two of the first, second, or third spectrums of light received from the single lens;
a memory element (329) for storing computer executable instructions; and,
at least one processor (330) configured to execute the computer executable instructions to generate, using the data, at least two of first, second, or third images of the first, second, and third portions, respectively.

2. The optical imaging system (102) of claim 1, wherein:
the first portion includes a drop pendant (338) from the drip tube (110) and the first image includes an image of the drop;
the at least one specially programmed processor (330) is configured to determine a volume of the pendant drop using the first image;
the third portion includes a meniscus (146) for fluid in the drip chamber (106) and the third image includes an image of the meniscus; and,
the at least one processor configured to execute the computer executable instructions to determine a position of the meniscus using the third image.

3. The optical imaging system (102) of claim 1, wherein an air bubble (344) is present in the second portion and the second image includes an image of the air bubble, and the at least one processor (330) is configured to execute the computer executable instructions to determine a volume of the air bubble using the second image.

4. The optical imaging system (102) of claim 1, wherein:
the at least one light source emits at least two of red, blue, and green spectrum light;
the first spectrum of light consists of one of the red, blue, or green spectrum light;
the second spectrum of light consists of one of the red, blue, or green spectrum light not included in the first spectrum; and,
the third spectrum of light consists of one of the red, blue, or green spectrums of light not included in the first or second spectrums of light.

5. The optical imaging system (102) of claim 1, wherein the optics system (319) includes a mirror (192) for reflecting one only of the first, second, or third spectrums of light transmitted by the first, second, or third portions, respectively.

6. The optical imaging system (102) of claim 1, wherein the optics system (319) includes:
a first mirror (346) for reflecting one only of the first, second, or third spectrums of light transmitted by the first, second, or third portions, respectively; and,
a second mirror (346A) for reflecting another only of the first, second, or third spectrums of light transmitted by the first, second, or third portions, respectively.

7. The optical imaging system (102) of claim 1, wherein the optics system (319) includes a beam combiner (348A) for reflecting two only of the first, second, or third spectrums of light transmitted by the first, second, or third portions, respectively.

8. The optical imaging system (102) of claim 1, wherein the optics system (319) includes a mirror (346) for:
transmitting to one of the first, second, or third portions, one only of the first, second, or third spectrums of light emitted by the at least one light source (122); or, reflecting to one of the first, second, or third portions, one only of the first, second, or third spectrums of light emitted by the at least one light source.

9. A method of imaging an infusion tube (302) having a drip chamber (304) including a first portion with a drip tube (308), a second portion with an exit port (312), and a third portion located between the first and second portions, **characterized in that** the method further comprises the steps of:
storing, in a memory element (329), computer executable instructions;
emitting at least two of the first, second, or third spectrums of light from at least one light source (318);
receiving and transmitting, using a single lens (320) at least two of:
the first spectrum of light transmitted through the first portion;
the second spectrum of light transmitted through the second portion; or,
the third spectrum of light transmitted through the third portion;
receiving, using a single image sensor (322), the at least two of the first, second, or third spectrums of light from the single lens;
generating and transmitting, using the single image sensor, data characterizing the at least two of the first, second, or third spectrums of light received from the single lens; and,
executing, using the at least one processor (330), the computer executable instructions to generate, using the data, at least two of first, second, or third images of the first, second, and third portions, respectively.

10. The method of claim 9, wherein:
the first portion includes a drop pendant (338) from the drip tube (308) and the first image includes an image of the drop; and,
the third portion includes a meniscus (342) for fluid in the drip chamber (304) and the third image includes an image of the meniscus; and,
the method further comprising executing, using the at least one processor (330), the computer executable instructions to:
determine, using the first image, a volume of the pendant drop using the first image; and,
determining, using the third image, a position of the meniscus using the third image.

11. The method of claim 9, wherein an air bubble (344) is present in the second portion and the second image includes an image of the air bubble, and further comprising executing, using the at least one processor (330), the computer executable instructions to determine, using the second image, a volume of the air bubble.

12. The method of claim 9, wherein:
emitting at least two of the first, second, or third spectrums of light includes emitting at least two of red, blue, or green spectrum light;
the first spectrum of light consists of one of the red, blue, or green spectrum light;
the second spectrum of light consists of one of the red, blue, or green spectrum light not included in the first spectrum; and,
the third spectrum of light consists of one of the red, blue, or green spectrums of light not included in the first or second spectrums of light.

13. The method of claim 9, further comprising reflecting, using a mirror (346), one only of the first, second, or third spectrums of light transmitted by the first, second, or third portions, respectively.

14. The method of claim 9, further comprising:
reflecting, using a first mirror (346), one only of the first, second, or third spectrums of light transmitted by the first, second, or third portions, respectively;
reflecting, using a second mirror (346A), another only of the first, second, or third spectrums of light transmitted by the first, second, or third portions, respectively; and reflecting, using a beam combiner (348A), two only of the first, second, or third spectrums of light transmitted by the first, second, or third portions, respectively.

15. The method of claim 9, further comprising:
transmitting, using a mirror (346), to one of the first, second, or third portions, one only of the first, second, or third spectrums of light emitted by the at least one light source; or, reflecting, using a mirror (346A), to one of the first, second, or third portions, one only of the first, second, or third spectrums of light emitted by the at least one light source.

## Patentansprüche

1. Optisches Bildgebungssystem (102) zur Verwendung mit einem Infusionsschlauch, der eine Tropfkammer (106) mit einem ersten Abschnitt mit einem Tropfschlauch (110), einem zweiten Abschnitt mit einer Austrittsöffnung (312) und einem zwischen dem ersten und dem zweiten Abschnitt liegenden dritten Abschnitt aufweist, wobei das optische Bildgebungssystem **dadurch gekennzeichnet ist, dass** es ferner Folgendes umfasst:
mindestens eine Lichtquelle (122) zum Ausstrahlen von mindestens zweien aus einem ersten, einem zweiten und einem dritten Lichtspektrum,
ein Optiksystem (319), das Folgendes enthält:
eine einzelne Linse (148) zum Empfangen und Durchlassen von mindestens zweien aus:
dem ersten Lichtspektrum, das von dem ersten Abschnitt durchgelassen wird,
dem zweiten Lichtspektrum, das von dem zweiten Abschnitt durchgelassen wird, und
dem dritten Lichtspektrum, das von dem dritten Abschnitt durchgelassen wird,
einen einzelnen Bildsensor (322) zum:
Empfangen der mindestens zwei aus dem ersten, dem zweiten und dem dritten Lichtspektrum von der einzelnen Linse und
Erzeugen und Übertragen von Daten, die die mindestens zwei aus dem ersten, dem zweiten und dem dritten Lichtspektrum kennzeichnen, die von der einzelnen Linse empfangen werden,
ein Speicherelement (329) zum Speichern von per Computer ausführbaren Anweisungen und
mindestens einen Prozessor (330), der so konfiguriert ist, dass er die per Computer ausführbaren Anweisungen ausführt, um unter Verwendung der Daten mindestens zwei aus einem ersten, einem zweiten und einem dritten Bild des ersten, des zweiten beziehungsweise des dritten Abschnitts zu erzeugen.

2. Optisches Bildgebungssystem (102) nach Anspruch 1, wobei:
der erste Abschnitt einen an dem Tropfschlauch (110) hängenden Tropfen (338) enthält und das erste Bild ein Bild von dem Tropfen enthält,
der mindestens eine speziell programmierte Prozessor (330) so konfiguriert ist, dass er unter Verwendung des ersten Bilds ein Volumen des hängenden Tropfens bestimmt,
der dritte Abschnitt einen Meniskus (146) für Fluid in der Tropfkammer (106) enthält und das dritte Bild ein Bild von dem Meniskus enthält und
der mindestens eine Prozessor so konfiguriert ist, dass er die per Computer ausführbaren Anweisungen ausführt, um unter Verwendung des dritten Bilds eine Position des Meniskus zu bestimmen.

3. Optisches Bildgebungssystem (102) nach Anspruch 1, wobei sich in dem zweiten Abschnitt eine Luftblase (344) befindet und das zweite Bild ein Bild von der Luftblase enthält und der mindestens eine Prozessor (330) so konfiguriert ist, dass er die per Computer ausführbaren Anweisungen ausführt, um unter Verwendung des zweiten Bilds ein Volumen der Luftblase zu bestimmen.

4. Optisches Bildgebungssystem (102) nach Anspruch 1, wobei:
die mindestens eine Lichtquelle mindestens zwei aus Licht im roten, im blauen und im grünen Spektrum ausstrahlt,
das erste Lichtspektrum aus Licht im roten, im blauen oder im grünen Spektrum besteht,
das zweite Lichtspektrum aus Licht im roten, im blauen oder im grünen Spektrum besteht, das nicht im ersten Spektrum enthalten ist, und
das dritte Lichtspektrum aus Licht im roten, im blauen oder im grünen Spektrum besteht, das nicht im ersten oder im zweiten Lichtspektrum enthalten ist.

5. Optisches Bildgebungssystem (102) nach Anspruch 1, wobei das Optiksystem (319) einen Spiegel (192) zum Reflektieren nur des ersten, des zweiten oder des dritten Lichtspektrums enthält, das von dem ersten, dem zweiten beziehungsweise dem dritten Abschnitt durchgelassen wird.

6. Optisches Bildgebungssystem (102) nach Anspruch 1, wobei das Optiksystem (319) Folgendes enthält:
einen ersten Spiegel (346) zum Reflektieren nur des ersten, des zweiten oder des dritten Lichtspektrums, das von dem ersten, dem zweiten beziehungsweise dem dritten Abschnitt durchgelassen wird, und
einen zweiten Spiegel (346A) zum Reflektieren nur eines anderen aus dem ersten, dem zweiten und dem dritten Lichtspektrum, das von dem ersten, dem zweiten beziehungsweise dem dritten Abschnitt durchgelassen wird.

7. Optisches Bildgebungssystem (102) nach Anspruch 1, wobei das Optiksystem (319) einen Strahlvereiniger (348A) zum Reflektieren von nur zweien aus dem ersten, dem zweiten und dem dritten Lichtspektrum enthält, die von dem ersten, dem zweiten beziehungsweise dem dritten Abschnitt durchgelassen werden.

8. Optisches Bildgebungssystem (102) nach Anspruch 1, wobei das Optiksystem (319) einen Spiegel (346) für Folgendes enthält:
zum Übertragen nur des ersten, des zweiten oder des dritten von der mindestens einen Lichtquelle (122) ausgestrahlten Lichtspektrums zu dem ersten, dem zweiten oder dem dritten Abschnitt oder
zum Reflektieren nur des ersten, des zweiten oder des dritten von der mindestens einen Lichtquelle ausgestrahlten Lichtspektrums zu dem ersten, dem zweiten oder dem dritten Abschnitt.

9. Verfahren zum Abbilden eines Infusionsschlauchs (302), der eine Tropfkammer (304) mit einem ersten Abschnitt mit einem Tropfschlauch (308), einem zweiten Abschnitt mit einer Austrittsöffnung (312) und einem zwischen dem ersten und dem zweiten Abschnitt liegenden dritten Abschnitt aufweist, **dadurch gekennzeichnet, dass** das Verfahren ferner die folgenden Schritte umfasst:
Speichern von per Computer ausführbaren Anweisungen in einem Speicherelement (329),
Ausstrahlen von mindestens zweien aus einem ersten, einem zweiten und einem dritten Lichtspektrum von mindestens einer Lichtquelle (318),
unter Verwendung einer einzelnen Linse (320) Empfangen und Durchlassen mindestens zweier aus:
dem ersten Lichtspektrum, das von dem ersten Abschnitt durchgelassen wird,
dem zweiten Lichtspektrum, das von dem zweiten Abschnitt durchgelassen wird, und
dem dritten Lichtspektrum, das von dem dritten Abschnitt durchgelassen wird,
Empfangen der mindestens zwei aus dem ersten, dem zweiten und dem dritten Lichtspektrum von der einzelnen Linse unter Verwendung eines einzelnen Bildsensors (322),
Erzeugen und Übertragen von Daten, die die mindestens zwei aus dem ersten, dem zweiten und dem dritten Lichtspektrum kennzeichnen, die von der einzelnen Linse empfangen werden, unter Verwendung des einzelnen Bildsensors und
Ausführen der per Computer ausführbaren Anweisungen unter Verwendung des mindestens einen Prozessors (330) zum Erzeugen von mindestens zwei aus einem ersten, einem zweiten und einem dritten Bild des ersten, des zweiten beziehungsweise des dritten Abschnitts unter Verwendung der Daten.

10. Verfahren nach Anspruch 9, wobei:
der erste Abschnitt einen an dem Tropfschlauch (308) hängenden Tropfen (338) enthält und das erste Bild ein Bild von dem Tropfen enthält und
der dritte Abschnitt einen Meniskus (342) für Fluid in der Tropfkammer (304) enthält und das dritte Bild ein Bild von dem Meniskus enthält und
das Verfahren ferner das Ausführen der per Computer ausführbaren Anweisungen unter Verwendung des mindestens einen Prozessors (330) umfasst zum:
Bestimmen, unter Verwendung des ersten Bilds, eines Volumens des hängenden Tropfens unter Verwendung des ersten Bilds und
Bestimmen, unter Verwendung des dritten Bilds, einer Position des Meniskus unter Verwendung des dritten Bilds.

11. Verfahren nach Anspruch 9, wobei sich in dem zweiten Abschnitt eine Luftblase (344) befindet und das zweite Bild ein Bild von der Luftblase enthält und das ferner das Ausführen der per Computer ausführbaren Anweisungen unter Verwendung des mindestens einen Prozessors (330) zum Bestimmen eines Volumens der Luftblase unter Verwendung des zweiten Bilds umfasst.

12. Verfahren nach Anspruch 9, wobei:
das Ausstrahlen mindestens zweier aus dem ersten, dem zweiten und dem dritten Lichtspektrum das Ausstrahlen von Licht in mindestens zweien aus dem roten, dem blauen und dem grünen Spektrum enthält,
das erste Lichtspektrum aus Licht im roten, im blauen oder im grünen Spektrum besteht,
das zweite Lichtspektrum aus Licht im roten, im blauen oder im grünen Spektrum besteht, das nicht im ersten Spektrum enthalten ist, und
das dritte Lichtspektrum aus Licht im roten, im blauen oder im grünen Spektrum besteht, das nicht im ersten oder im zweiten Lichtspektrum enthalten ist.

13. Verfahren nach Anspruch 9, das ferner das Reflektieren nur des ersten, des zweiten oder des dritten Lichtspektrums, das von dem ersten, dem zweiten beziehungsweise dem dritten Abschnitt durchgelassen wird, unter Verwendung eines Spiegels (346) umfasst.

14. Verfahren nach Anspruch 9, das ferner Folgendes umfasst:
Reflektieren nur des ersten, des zweiten oder des dritten Lichtspektrums, das von dem ersten, dem zweiten beziehungsweise dem dritten Abschnitt durchgelassen wird, unter Verwendung eines ersten Spiegels (346),
Reflektieren nur eines anderen aus dem ersten, dem zweiten und dem dritten Lichtspektrum, das von dem ersten, dem zweiten beziehungsweise dem dritten Abschnitt durchgelassen wird, unter Verwendung eines zweiten Spiegels (346A) und
Reflektieren von nur zweien aus dem ersten, dem zweiten und dem dritten Lichtspektrum, die von dem ersten, dem zweiten beziehungsweise dem dritten Abschnitt durchgelassen werden, unter Verwendung eines Strahlvereinigers (348A).

15. Verfahren nach Anspruch 9, das ferner Folgendes umfasst:
Übertragen nur des ersten, des zweiten oder des dritten von der mindestens einen Lichtquelle ausgestrahlten Lichtspektrums zu dem ersten, dem zweiten oder dem dritten Abschnitt unter Verwendung eines Spiegels (346) oder Reflektieren nur des ersten, des zweiten oder des dritten von der mindestens einen Lichtquelle ausgestrahlten Lichtspektrums zu dem ersten, dem zweiten oder dem dritten Abschnitt unter Verwendung eines Spiegels (346A).

## Revendications

1. Système d'imagerie optique (102) destiné à être utilisé avec un tube de perfusion présentant une chambre compte-gouttes (106) comprenant une première partie ayant un tube compte-gouttes (110), une deuxième partie ayant un orifice de sortie (312), et une troisième partie située entre les première et deuxième parties, le système d'imagerie optique étant **caractérisé en ce qu'**il comporte en outre :
au moins une source de lumière (122) destinée à émettre au moins l'un parmi un premier, un deuxième ou un troisième spectre de lumière ;
un système optique (319) comprenant :
une seule lentille (148) destinée à recevoir et à transmettre au moins deux parmi :
le premier spectre de lumière transmis à travers la première partie ;
le deuxième spectre de lumière transmis à travers la deuxième partie ; ou,
le troisième spectre de lumière transmis à travers la troisième partie ;
un seul capteur d'images (322) destiné à :
recevoir lesdits au moins deux parmi les premier, deuxième ou troisième spectres de lumière depuis la seule lentille ; et,
générer et transmettre des données caractérisant lesdits au moins deux parmi les premier, deuxième ou troisième spectres de lumière reçus depuis la seule lentille ;
un élément de mémoire (329) destiné à stocker des instructions exécutables par ordinateur ; et,
au moins un processeur (330) configuré pour exécuter les instructions exécutables par ordinateur pour générer, en utilisant les données, au moins deux parmi les première, deuxième ou troisième images des première, deuxième et troisième parties respectives.

2. Système d'imagerie optique (102) selon la revendication 1, dans lequel :
la première partie comprend une goutte (338) suspendue depuis le tube compte-gouttes (110) et la première image comprend une image de la goutte ;
ledit au moins un processeur spécialement programmé (330) est configuré pour déterminer un volume de la goutte suspendue en utilisant la première image ;
la troisième partie comprend un ménisque (146) pour le fluide dans la chambre compte-gouttes (106) et la troisième image comprend une image du ménisque ; et,
ledit au moins un processeur est configuré pour exécuter les instructions exécutables par ordinateur afin de déterminer une position du ménisque en utilisant la troisième image.

3. Système d'imagerie optique (102) selon la revendication 1, dans lequel une bulle d'air (344) est présente dans la deuxième partie et la deuxième image comprend une image de la bulle d'air, et ledit au moins un processeur (330) est configuré pour exécuter les instructions exécutables par ordinateur afin de déterminer un volume de la bulle d'air en utilisant la deuxième image.

4. Système d'imagerie optique (102) selon la revendication 1, dans lequel :
ladite au moins une source de lumière émet au moins deux parmi une lumière de spectre rouge, bleue et verte ;
le premier spectre de lumière est constitué de l'une parmi la lumière de spectre rouge, bleue ou verte ;
le deuxième spectre de lumière est constitué de l'une parmi la lumière de spectre rouge, bleue ou verte qui n'est pas comprise dans le premier spectre ; et,
le troisième spectre de lumière est constitué de l'un parmi le spectre de lumière rouge, bleu ou vert qui n'est pas compris dans le premier ou le deuxième spectre de lumière.

5. Système d'imagerie optique (102) selon la revendication 1, dans lequel le système optique (319) comprend un miroir (192) destiné à réfléchir l'un seulement parmi les premier, deuxième ou troisième spectres de lumière transmis respectivement par les première, deuxième ou troisième parties.

6. Système d'imagerie optique (102) selon la revendication 1, dans lequel le système optique (319) comprend :
un premier miroir (346) destiné à réfléchir l'un seulement parmi les premier, deuxième ou troisième spectres de lumière transmis respectivement par les première, deuxième ou troisième parties; ; et,
un deuxième miroir (346A) destiné à réfléchir un autre seulement parmi les premier, deuxième ou troisième spectres de lumière transmis respectivement par les première, deuxième ou troisième parties.

7. Système d'imagerie optique (102) selon la revendication 1, dans lequel le système optique (319) comprend un combinateur de faisceaux (348A) destiné à réfléchir deux seulement parmi les premier, deuxième ou troisième spectres de lumière transmis respectivement par les première, deuxième ou troisième parties.

8. Système d'imagerie optique (102) selon la revendication 1, dans lequel le système optique (319) comprend un miroir (346) destiné à :
transmettre à l'une parmi les première, deuxième ou troisième parties, l'un seulement parmi les premier, deuxième ou troisième spectres de lumière émis par la ladite au moins une source de lumière (122) ; ou,
réfléchir vers l'une parmi les première, deuxième ou troisième parties, l'un seulement parmi les premier, deuxième ou troisième spectres de lumière émis par la ladite au moins une source de lumière.

9. Procédé d'imagerie d'un tube de perfusion (302) présentant une chambre compte-gouttes (304) comprenant une première partie ayant un tube compte-gouttes (308), une deuxième partie ayant un orifice de sortie (312), et une troisième partie située entre les première et deuxième parties, **caractérisé en ce que**
le procédé comprend en outre les étapes consistant à :
stocker, dans un élément de mémoire (329), des instructions exécutables par ordinateur ;
émettre au moins deux parmi les premier, deuxième ou troisième spectres de lumière depuis au moins une source de lumière (318) ;
recevoir et transmettre, en utilisant une seule lentille (320) au moins deux parmi :
le premier spectre de lumière transmis à travers la première partie ;
le deuxième spectre de lumière transmis à travers la deuxième partie ; ou,
le troisième spectre de lumière transmis à travers la troisième partie ;
recevoir, en utilisant un seul capteur d'images (322), lesdits au moins deux parmi les premier, deuxième ou troisième spectres de lumière depuis la seule lentille ;
générer et transmettre en utilisant ledit capteur d'images unique, des données caractérisant lesdits au moins deux parmi les premier, deuxième ou troisième spectres de lumière reçus depuis la seule lentille ; et,
exécuter, en utilisant ledit au moins un processeur (330), les instructions exécutables par ordinateur pour générer, en utilisant les données, au moins deux parmi les première, deuxième ou troisième images des première, deuxième et troisième parties respectives.

10. Procédé selon la revendication 9, dans lequel :
la première partie comprend une goutte (338) suspendue depuis le tube compte-gouttes (308) et la première image comprend une image de la goutte ; et,
la troisième partie comprend un ménisque (342) pour le fluide dans la chambre compte-gouttes (304) et
la troisième image comprend une image du ménisque ; et,
le procédé comprenant en outre l'exécution, en utilisant ledit au moins un processeur (330), des instructions exécutables par ordinateur afin de :
déterminer, en utilisant la première image, un volume de la goutte suspendue en utilisant la première image ; et
déterminer, en utilisant la troisième image, une position du ménisque en utilisant la troisième image.

11. Procédé selon la revendication 9, dans lequel une bulle d'air (344) est présente dans la deuxième partie et la deuxième image comprend une image de la bulle d'air, et comprend en outre l'exécution, en utilisant ledit au moins un processeur (330), des instructions exécutables par ordinateur afin de déterminer, en utilisant la deuxième image, un volume de la bulle d'air.

12. Procédé selon la revendication 9, dans lequel :
l'émission d'au moins deux parmi les premier, deuxième ou troisième spectres de lumière comprend l'émission d'au moins deux parmi la lumière de spectre rouge, bleue ou verte ;
le premier spectre de lumière est constitué de l'une parmi la lumière de spectre rouge, bleue ou verte ;
le deuxième spectre de lumière est constitué de l'une parmi la lumière de spectre rouge, bleue ou verte qui n'est pas comprise dans le premier spectre ; et,
le troisième spectre de lumière est constitué de l'un parmi le spectre de lumière rouge, bleu ou vert qui n'est pas compris dans le premier ou le deuxième spectre de lumière.

13. Procédé selon la revendication 9, comprenant en outre l'étape consistant à réfléchir, en utilisant un miroir (346), l'un seulement parmi les premier, deuxième ou troisième spectres de lumière transmis respectivement par les première, deuxième ou troisième parties.

14. Procédé selon la revendication 9, comprenant en outre :
le réfléchissement, en utilisant un premier miroir (346), de l'un seulement parmi les premier, deuxième ou troisième spectres de lumière transmis respectivement par les première, deuxième ou troisième parties ;
le réfléchissement, en utilisant le deuxième miroir (346A), d'un autre seulement parmi les premier, deuxième ou troisième spectres de lumière transmis respectivement par les première, deuxième ou troisième parties ; et
le réfléchissement, en utilisant un combinateur de faisceaux (348A), de deux seulement parmi les premier, deuxième ou troisième spectres de lumière transmis respectivement par les première, deuxième ou troisième parties.

15. Procédé selon la revendication 9, comprenant en outre :
la transmission, en utilisant un miroir (346), à l'une parmi les première, deuxième ou troisième parties, de l'un seulement parmi les premier, deuxième ou troisième spectres de lumière émis par ladite au moins une source de lumière ; ou, le réfléchissement, en utilisant un miroir (346A), vers l'une parmi les première, deuxième ou troisième parties, de l'un seulement parmi les premier, deuxième ou troisième spectres de lumière émis par ladite au moins une source de lumière.
